(19) Europäisches Patentamt
European Patent Office
Office européen des brevets

(11) EP 4 732 950 A1

(12) EUROPEAN PATENT APPLICATION

(43) Date of publication:
29.04.2026 Bulletin 2026/18

(21) Application number: 25166456.1

(22) Date of filing: 26.03.2025

(51) International Patent Classification (IPC):
B01L 3/00 (2006.01)

(52) Cooperative Patent Classification (CPC):
B01L 3/5023; B01L 3/502707; B01L 3/50273;
B01L 3/502738; B01L 3/502746; B01L 3/527;
B01L 2200/025; B01L 2200/027; B01L 2200/12;
B01L 2200/16; B01L 2300/0816; B01L 2300/0858;
B01L 2300/0867; B01L 2300/087; B01L 2300/0883;
(Cont.)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 22.10.2024 CN 202411483284
31.10.2024 US 202463714264 P
10.03.2025 CN 202510279542

(71) Applicant: Hangzhou Biotest Biotech Co., Ltd.
Yuhang District
Hangzhou (CN)

(72) Inventors:
• ZHANG, Zhenxing
Hangzhou (CN)
• WU, Shujiang
Hangzhou (CN)

(74) Representative: Piotrowicz, Pawel Jan Andrzej et al
Venner Shipley LLP
406 Cambridge Science Park
Milton Road
Cambridge CB4 0WW (GB)

(54) **MICROFLUIDIC DEVICE AND METHOD FOR TESTING ANALYTE IN LIQUID SAMPLE**

(57) The invention provides a microfluidic device, including: a fluid channel including an inlet and an outlet; and a dried reagent area and a testing area that are located in the channel. The testing area is located downstream of the dried reagent area. The outlet of the fluid channel is provided in communication with a pump. The inlet of the fluid channel is configured to receive a fluid sample.

Fig. 2

**(Cont. next page)**

(52) Cooperative Patent Classification (CPC): (Cont.)
B01L 2300/165; B01L 2400/0406;
B01L 2400/0688; B01L 2400/086; B01L 2400/088

**Description**

CROSS-REFERENCE TO RELATED APPLICATIONS

The invention claims priorities to Chinese Patent Application No. 2024114832844, filed on October 22, 2024 ;2025102795425, filed on March 10, 2025 and US Provisional Application No. 63/714,264, filed on October 31, 2024, the abstract, specification, claims and accompanying drawings of which are incorporated herein by reference in their entirety as part of this application.

BACKGROUND OF THE INVENTION

Field of the Invention

The invention belongs to the field of diagnosis, and in particular to testing of analytes in a liquid sample by using microfluidic channels in the field of rapid diagnosis.

Description of the Related Art

The following description of the background art is merely a description of some background common knowledge, and does not limit the invention in any way.

At present, test devices for testing the presence or absence of an analyte in a sample are widely used in hospitals or homes, and such test devices for rapid diagnosis include one or more test strips, for example, for early pregnancy testing and drug of abuse testing. Such test devices for rapid diagnosis are very convenient, and can obtain a test result from the test strips in one minute or at most ten minutes or so.

Microfluidics involves manipulating a small volume of one or more fluids, such as gases and/or liquids. The total volume of the fluids may be, for example, about 250 microliters or less, such as about 125 microliters or less, about 75 microliters or less, about 50 microliters or less, or about 25 microliters or less. In conventional detection, it is particularly desired that micro-samples can be accurately tested, such that one or more indicators can be accomplished with only a small number of samples without deliberately pursuing the volume or number of samples. For example, Chinese patent application No. 202110956861.7 discloses a microfluidic device, which is provided with a filter to treat the sample in advance. However, the filter is complicated in structure and high in manufacturing cost, and is difficult to realize in real products. It is known to use microfluidics to determine the presence of at least one target in a liquid specimen. For example, U.S. Pat. No. 7,824,611 and PCT/US2013/035505 disclose immunoassay devices, assay systems and device components, including at least two opposing surfaces disposed a capillary distance apart, at least one of which is capable of immobilizing at least one target ligand or a conjugate in an amount related to the presence or amount of target ligand in the sample from a fluid sample in a zone for controlled fluid movement to, through or away the zone. U.S. Pat. No. 7,824,611 further discloses use of reagents (such as receptors and conjugates) and biosensors (such as electrochemical, optical, electro-optical or acoustic/mechanical devices) to determine the presence of one or more targets.

For another example, Chinese patent application No. 202310561939.4 discloses a microfluidic device. However, this device only briefly introduces the general design, and needs to realize the test of microliquid, which is still not perfect and cannot achieve many functions.

In view of the traditional technical problems above, it is necessary to make improvements and provide a new test for microfluidic liquid samples that can detect at least one analyte in the liquid sample by using one capillary channel.

BRIEF SUMMARY OF THE INVENTION

In order to solve some problems in the traditional technology, the invention provides a microfluidic device, including a channel containing a liquid inlet and an outlet. A dried reagent area and a testing area are arranged in the channel. As a liquid sample flows in the channel, the test or assay of an analyte in the liquid sample can be completed. The device makes the test more convenient and the result more accurate, and requires a very small amount of liquid sample.

Therefore, in one aspect, the invention provides a microfluidic device, including a channel. The channel has an inlet and an outlet. The inlet is configured to allow a liquid sample to enter, and the outlet is the direction for the liquid to flow from the inlet to the outlet. In some embodiments, some areas in the channel have capillary force, and the liquid may flow by the capillary force. In some embodiments, some areas in the channel have capillary force, or different areas have different capillary actions. In this way, the velocity of the liquid flowing in different areas of the channel can be different, if only by capillary force. Optionally, the channel does not have capillary force, and the liquid flows in the channel by the power of a pump at the outlet. In some embodiments, in the channel, the liquid flows in the channel by capillary force in combination with the action of the pump.

In some embodiments, a dried reagent area is arranged downstream of the inlet in the channel. A first receptor is immobilized in the dried reagent area. The first receptor may specifically bind to the analyte in the liquid sample. In some embodiments, the dried reagent area is close to the inlet. In some embodiments, the first receptor may be marked by a marker. Dried reagents include the immobilized first receptor, and can be dissolved by the liquid sample and flow along with the liquid sample. In some embodiments, a flow velocity of the liquid in the dried reagent area changes, for example, gradually increases or gradually decreases. In some embodiments, the flow may depend on the capillary force of the channel, or the flow velocity of the liquid may change with the changing capillary force. In some embodiments, the capillary force of the inlet of the dried reagent area is less than the capillary force of the outlet of the dried reagent area, and the capillary force decreases or increases gradually from the inlet to the outlet. In some embodiments, a width of the inlet of the dried reagent area is greater than a width of the outlet, or a cross-sectional area of the inlet of the dried reagent area is greater than a cross-sectional area of the outlet, or a radius of the inlet of the dried reagent area is greater than a radius or diameter of the outlet. In some embodiments, the liquid flows in the dried reagent area only by the capillary force of the dried reagent area. In some optional embodiments, of course, the flow may not depend on the capillary force, but on subatmospheric or negative pressure formed in the channel by gas extraction by a suction pump in communication with the outlet. Of course, the flow of the liquid may also depend on the combined action of the capillary force of the channel and the pump.

In some embodiments, a sample application channel is arranged upstream of the dried reagent area, and the sample application channel is in fluid communication with the dried reagent area. In some embodiments, the capillary force of the sample channel is less than the capillary force of the dried reagent area. In some embodiments, a height of the sample channel is greater than a height of the inlet of the dried reagent area. In some embodiments, the outlet of the sample channel overlaps the inlet of the dried reagent area. In some embodiments, the inlet of the sample channel is in fluid communication with the sample application opening, and the sample flows into the sample channel through the sample application opening. In some embodiments, the sample channel includes a vent hole, or the device further includes a vent hole. The vent hole is in fluid communication with the sample channel, or the vent hole is located in the channel between the sample application opening and the inlet of the dried reagent area and is in communication with outside atmosphere.

In some embodiments, a choke area is arranged downstream of the dried reagent area in the channel. The choke area functions to retard or slow the flow of the liquid sample entering from the inlet of the dried reagent area to the downstream of the dried reagent area, or retard or slow the flow of the liquid from the dried reagent area to the downstream, so that the liquid can stay in the dried reagent area for a long time and the dried reagents in the area can be moistened or dissolved by the liquid (e.g., the liquid sample) or separated from the dried reagent area. The capillary force of the dried reagent area is greater than the capillary force of the choke area, or the capillary force of the choke area is less than the capillary force of the dried reagent area, so that the liquid flows faster at a place where the capillary force is larger and slower at a place where the capillary force is smaller. When the liquid flows from a place where the capillary force is larger to a place where the capillary force is smaller, it will flow slower naturally, thereby reducing the flow velocity of the liquid. This can allow the liquid sample to stay in the dried reagent area for a longer time so as to contact the reagents thereon, so that more dried reagents can be dissolved during the flow process of the liquid. In some embodiments, the liquid sample completely dissolves the reagents (the first receptor and the marker or other reagents) in the dried reagent area. Alternatively, a flow velocity of the liquid in the dried reagent area is greater than a flow velocity of the liquid in the choke area. The retarding of the flow is caused by the action of the choke area, such as the decrease in capillary force in the choke area. In some embodiments, the capillary force of a part of the choke area is less than the capillary force of the outlet of the dried reagent area. In some embodiments, when the height of the channel is constant, a maximum width of the choke area is greater than the width of the outlet of the dried reagent area, for example, 1.5, 1.5 or 2 times the width of the outlet.

In some embodiments, a mixing area is arranged downstream of the dried reagent area or downstream of the choke area, and the liquid sample and the dried reagents dissolved are mixed to react thoroughly in the mixing area. In some embodiments, the choke area is arranged between the mixing area and the dried reagent area, or the choke area connects the dried reagent area with the mixing area. In some embodiments, the choke area has an inlet and an outlet. In some embodiments, the dried reagent area has an inlet and an outlet, and the outlet of the dried reagent area is sometimes the inlet of the choke area, or the inlet of the choke area is a very short distance from the outlet of the dried reagent area. Similarly, the mixing area has an inlet and an outlet, and in some embodiments, the inlet of the mixing area is the outlet of the choke area, or the outlet of the choke area is a short distance from the inlet of the mixing area, for example, 1-2 millimeters or 10--2 $\mu$m. In some embodiments, a transition area is arranged between the outlet of the choke area and the inlet of the mixing area, and through the transition area, a flow direction of the liquid or a flow velocity of the liquid is changed. In some embodiments, a cross-sectional area of the inlet of the choke area is greater than that of the outlet of the dried reagent area, or a maximum inner diameter of the choke area is greater than that of the outlet of the dried reagent area, so that a cross-sectional area at the outlet increases naturally and the capillary force is reduced. Thereby, the flow velocity of the liquid flowing from the dried reagent area is reduced due to the capillary force. In some embodiments, a maximum cross-sectional area of the choke area is greater than the cross-sectional area of the outlet of the dried reagent area, so that the capillary force is reduced. Of course, some hydrophobic reagents may also be arranged in the choke area to make the capillary force of the choke area smaller than the capillary force of the dried reagent area.

In some embodiments, resistance elements or division elements for changing flow properties of the liquid (such as the flow direction or velocity of the fluid) are arranged in the mixing area, so as to reduce the flow velocity of the liquid and/or (continuously) change the flow direction of the liquid in the channel where the mixing area is located, so that the liquid sample can thoroughly contact substances dissolved in the dried reagent area to thoroughly react in the mixing area. In some embodiments, a flow path of the liquid in the mixing area is S-shaped, Y-shaped or Y-shaped. The overall flow direction is from upstream to downstream, but the flow path is nonlinear. In some embodiments, the mixing area is nonlinear, so that the liquid also flows in the channel of the mixing area along a nonlinear path. In some embodiments, although the channel where the mixing area is located is linear, the liquid flows in the mixing area along a nonlinear and curved path, which looks like flowing in a linear channel. In some embodiments, the channel where the mixing area is located is curved, and the liquid also flows along a curved path in each of the mixing areas. The changes in the flow direction here refer to the specific changes in the flow direction instead of the linear flow under the condition that the overall flow direction of liquid remains unchanged. The mixing area includes one or more mixing units, and the liquid is divided and then gathered in the mixing unit. In some embodiments, the mixing unit includes one or more choke elements or division elements. The liquid is divided into 2 or more flow paths in the unit, and the 2 flow paths are gathered together and enter the next mixing unit. In some embodiments, flow velocities of different flow paths of the liquid may be the same or different, and distances of the flow paths may be the same or different. In some embodiments, the flow velocity changes with the changing capillary action of the flow path.

The cause of the specific changes in the flow direction is the resistance elements arranged in the mixing area. These resistance elements change the flow direction when the liquid flows through, but the liquid generally flows from the mixing area to the channel where the downstream testing area is located. In some embodiments, the resistance elements may be mechanical structures that are arranged in the channel where the mixing area is located. When the liquid meets these mechanical structures arranged, the flow path of the liquid will change. The structures may be posts and irregular disks, such as triangular, five-pointed star-shaped, rhombic and square protrusions. Actually, these structures are arranged in the channel where the mixing area is located to form one or more slits that allow the liquid to flow, and these slits are curved or twisted. Thus, when the liquid flows through these curved or twisted slits, the flow direction of the liquid changes partially, and the flow velocity also changes, so that the liquid can thoroughly contact and react with the dried reagents dissolved in the liquid. **In** some embodiments, some hydrophobic reagents may be arranged in the mixing area. These hydrophobic reagents have different hydrophobic abilities, so that the flow velocity of the liquid will also change when the liquid flows through areas having different hydrophobic abilities. Of course, in the mixing area, the size of the channel may also change. For example, in the mixing area, a section of the channel is small or thin, and then another section of the channel is large or thick. The small or thin section and the large or thick section appear alternately, so that the capillary force changes alternately. Thereby, the flow velocity of the liquid in such a channel changes alternately, thereby realizing the mixing function.

**In** some embodiments, the channel downstream of the mixing area is provided with one or a plurality of testing areas in which at least one second receptor is immobilized. These second receptors may specifically bind to the analyte (competitive method) or bind to a compound of the first receptor and the analyte. In some embodiments, the second receptor in these testing areas is immobilized and cannot flow with the liquid sample substantially. In some embodiments, the capillary force in the mixing area is greater than the capillary force of the liquid in the testing area, so that the flow velocity of the liquid in the mixing area is less than the flow velocity of the liquid in the testing area. The flow velocity depends solely on the capillary force of the channel. In some embodiments, a cross-sectional area of the outlet of the mixing area is less than a cross-sectional area of the testing area. In some embodiments, when the channel has a constant height, an inner diameter of the outlet of the mixing area is less than an inner diameter of the channel where the testing area is located, so that the capillary force is reduced near the outlet, thereby retarding the fluid in the mixing area from quickly flowing into the channel in the testing area.

In some embodiments, the testing area is arranged downstream of the mixing area. One or more testing areas are arranged in the channel where the testing area is located, and a second receptor is immobilized in each testing area. When there is one testing area, the second receptor is immobilized. When there are more testing areas, different second receptors are immobilized in the testing areas and configured to test different analytes in the liquid sample. These second receptors in the testing areas are immobilized and cannot flow with the liquid substantially. In some embodiments, the testing areas are arranged at intervals in sequence in the channel. In some embodiments, a transition area is arranged between the mixing area and the testing area. Through the transition area, the flow velocity of the liquid flowing out of the mixing area is reduced, or the flow properties are changed, so that a non-laminar flow is changed into a laminar flow. In some embodiments, the transition area changes the flow direction of the liquid.

In some embodiments, a waste liquid area is arranged downstream of the testing area. The waste liquid area is configured to collect the excess liquid. The liquid here may be the liquid sample, or the mixed liquid after the liquid sample flows through the dried reagent area, the mixing area and the testing area. In some embodiments, a depth of the channel where the waste liquid area is located is greater than a depth of other areas, or a width of the channel where the waste liquid area is located is greater than a width of other areas, so that the waste liquid area can accommodate a large volume of waste liquid.

In some embodiments, a sample application area, such as an application area, is arranged at the inlet of the channel. This application area has an opening through which the liquid sample is applied, and the applied liquid sample enters the channel through the inlet of the channel and enters the dried reagent area. A vent hole is arranged between the opening of the application area and the inlet of the channel, so that the liquid can smoothly enter the inlet of the channel to discharge air between the opening of the dried reagent area and the inlet of the channel. Once the liquid sample enters the inlet of the channel, it may flow in the dried reagent area by the capillary force of the channel.

In some embodiments, at the outlet of the channel, the outlet in fluid communication with the waste liquid area is connected to a pump, and the pump may extract gas or blow gas (pump gas) into the channel so as to cause the liquid to flow in the channel through the extraction or blowing of gas by the pump. In some embodiments, subatmospheric or negative pressure is formed in the channel by gas extraction by the pump, so as to drive the liquid in the channel to flow from upstream to downstream until to the waste liquid area.

In some embodiments, the flow of the liquid from the inlet of the channel through the dried reagent area to the choke area depends on the capillary force of the channel, or substantially or mainly depends on the capillary force of the channel. In this case, the flow may not depend on the extraction action of the pump.

In some embodiments, the flow of the liquid from the mixing area via the testing area to the waste liquid area substantially or mainly depends on the subatmospheric or negative pressure formed in the channel by the gas extraction of the pump. In some embodiments, under the gas extraction action of the pump, the flow of the liquid in the channel actually depends on the combined action of the capillary force of the channel and the subatmospheric or negative pressure formed by the gas extraction of the pump. In some embodiments, the flow of the liquid into the inlet of the channel and into the waste liquid area may solely depend on the capillary action of the channel, and certainly may also solely depend on the action of the pump.

In some embodiments, when the pump cooperates with the capillary channel to control the flow of the liquid, the liquid sample may be allowed to enter the channel along the flow direction, so that a part of the liquid sample completely and substantially dissolves the reagents in the dried reagent area and reacts in the testing area. Then, there is still a part of the liquid sample that contains little or no dried reagents. When this part of the liquid sample flows through the testing area, it can function to flush the testing area and take away some non-specific bindings, which makes the test more accurate and reduces non-specific reactions. Thus, in this way, by using the same liquid sample that is applied at one time, part of the liquid sample is used for assaying the analyte, and the other part is used for cleaning the testing area, which reduces the background. Therefore, in some embodiments, an excess of the sample is added to the inlet of the channel, the former liquid sample is allowed to dissolve the dried reagents to serve as a test sample, and the latter sample serves as an elution liquid to elute non-specific bindings or other impurities in the testing area, so that the liquid sample can perform two functions at the same time. Of course, the action of the pump can allow the liquid sample with the dried reagents to reciprocate in the testing area, so as to make the second receptor in the testing area thoroughly contact the liquid sample and react thoroughly. Of course, the flow velocity of the liquid in the channel where the testing area is located may be controlled. For example, in some embodiments, when the liquid enters the mixing area, the flow velocity of the liquid decreases, and the flow resistance increases. At this time, the pump is allowed to extract gas to increase the flow velocity of the liquid in the mixing area, thereby quickly completing the mixing of the liquid and allowing the liquid to enter the testing area for testing. In some embodiments, the gas extraction action of the pump is constant, that is, the gas extraction ability of the pump is substantially constant.

In some embodiments, a vent hole is further provided near the inlet of the channel. The vent hole is configured to discharge air in the channel under the action of the capillary force during the flow of the liquid after the liquid enters the inlet, so as to allow the liquid to flow smoothly. In some embodiments, the vent hole is provided between the inlet of the channel and the dried reagent area. In this way, when the liquid flows completely by the capillary force of the channel, if the inlet of the channel is blocked by the liquid, the excess gas can also be discharged through air ventilation so as to allow the liquid to smoothly flow downstream.

In some embodiments, the dried reagent area is a rough surface, so that the dried reagents can be attached to the rough surface without falling off easily. In some embodiments, in order to make the dried reagent the dried reagent solution attached to this area and also make the dried reagent solution easily dissolved by the liquid, a plurality of small posts are arranged in the dried reagent area, and microgaps are formed between the small posts. In this way, when the reagent-containing solution is added dropwise to the small posts, the liquid is distributed in the microgaps between the small posts. After the liquid is dried, the reagents are located in these gaps. Thus, when the liquid flows through the microgaps between the small posts, these dried reagents can be dissolved easily. In some embodiments, the dried reagent area is trapezoidal or flared in shape, with a large opening at one end and a small opening at the other end. The end with the large opening is connected to the sample application hole. When the liquid is added through the sample application hole, the liquid flows from the end with the large opening to the end with the small opening, so that the liquid also converges when flowing through the dried reagent area. In some embodiments, the end with the large opening is used as the inlet of the liquid sample, and the end with the small opening is used as the outlet of the liquid sample. When the liquid flows from the end with the small opening into the choke area, a width of the choke area is greater than that of the outlet of the dried reagent area, so that the capillary force is reduced, thereby retarding the liquid sample from flowing into the choke area and into the downstream mixing area.

In some embodiments, a filter element, such as filter paper, is arranged at the sample application hole and configured to remove interfering substances in the liquid sample, such as red blood cells in a blood sample.

In some embodiments, the dried reagent area is arranged in a recessed area in the channel, and a dried reagent, such as microspheres, is arranged in the recessed area. The microspheres of the dried reagent are provided or arranged in the recessed area, so that when the liquid flows through the recessed area, it can dissolve the dried microspheres to form a mixed solution. The recessed area is similar to a cavity, a cave or any other shape, and freeze-dried microspheres are arranged in these areas. The microspheres include a first receptor, and the first receptor may specifically bind to the analyte. In addition, the arrangement of the recessed area allows the liquid sample to enter and to dissolve the dried reagent, and also allows the liquid to flow downstream by the capillary force.

Beneficial effects

With the above structure, the sample can be accurately tested through one channel, and multiple analytes can be detected just by using a small amount of liquid sample.

BRIEF DESCRIPTION OF THE DRAWINGS

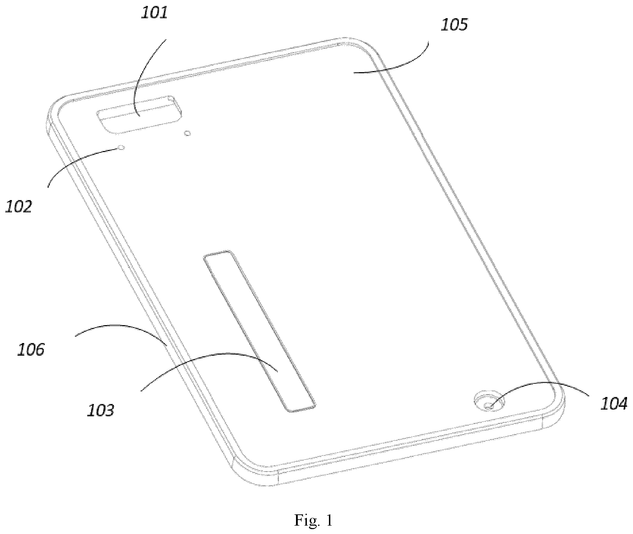

FIG. 1 is a schematic three-dimensional view of a microfluidic device after assembly in a specific implementation of the invention;
FIG. 2 is a schematic exploded structural view of the microfluidic device in a specific implementation of the invention;
FIG. 3 is a schematic three-dimensional structural view of a top cover (back side 1051) and a bottom plate (back side 1062) of the microfluidic device in a specific implementation of the invention;
FIG. 4 is a schematic structural view of the back side of the top cover of the microfluidic device in a specific embodiment of the invention;
FIG. 5 is a schematic partial sectional structural view of a combination of the bottom cover and the top cover of the microfluidic device in a specific embodiment of the invention;
FIG. 6 is a schematic exploded structural view of the top cover and the bottom plate, showing the positional relationship between a sample application channel and an inlet of the microchannel;
FIG. 7 is a schematic layout structural view of the channel of the bottom plate of the microfluidic device in a specific embodiment of the invention;
FIG. 8 is a schematic layout structural view of a dried reagent area, a mixing area and a testing area in a specific embodiment of the invention;
FIG. 9 is a schematic structural enlarged view of the dried reagent area in a specific embodiment of the invention;
FIG. 10A is a schematic partial structural enlarged view (mixing area) in a specific embodiment of the invention;
FIG. 10B is a structural top view of the mixing area in a specific embodiment of the invention;
FIG. 10C is a schematic partial structural enlarged view in a specific embodiment of the invention;
FIG. 11 is a schematic structural view of the dried reagent area and a choke area in a specific embodiment of the invention;
FIG. 12 is a schematic structural view showing transition between the mixing area and the testing area in a specific embodiment of the invention;
FIG. 13 is a schematic partial structural enlarged view of a cross-sectional area of the mixing area in a specific embodiment of the invention;
FIG. 14 is a schematic structural enlarged view of an area for immobilizing a reagent (reagent containing the first receptor) in the dried reagent area in a specific embodiment of the invention;

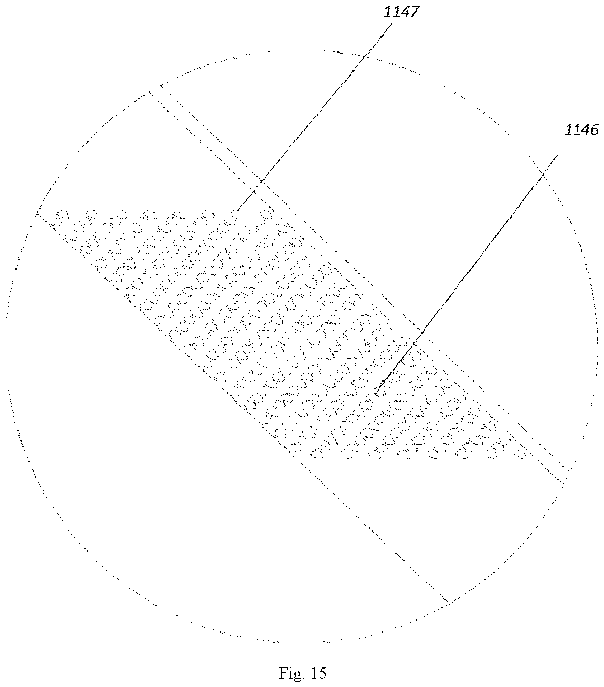

FIG. 15 is a schematic structural enlarged view of the testing area immobilized with a second receptor in a specific embodiment of the invention;
FIG. 16 is a schematic structural view of a waste liquid area and an outlet of the testing area in a specific embodiment of the invention;
FIG. 17 is a schematic structural view of the waste liquid area and an outlet of the microchannel in a specific embodiment of the invention;
FIG. 18 is a schematic view showing a specific test result of a test sample by using the test device in a specific embodiment of the invention (adding a blood sample to the sample application opening 101, and then reading the fluorescence on the testing unit 1144 in the testing area 114 through the window 103, which is the test result of CKMB 5ng/ml in the standard blood sample);
FIG. 19 is a schematic structural view of a microfluidic chip in a specific embodiment of the invention (16 is the serpentine mixing area);
FIG. 20A is a diagram of testing the flow velocity and time spent of the liquid in the testing area under different subatmospheric or negative pressures of the pump in an embodiment of the invention;

FIG. 20B is a diagram of testing the flow velocity and time spent of the liquid in the testing area under different subatmospheric or negative pressures of the pump in an embodiment of the invention;

FIG. 20C is a diagram of testing the flow velocity and time spent of the liquid in the testing area under different subatmospheric or negative pressures of the pump in an embodiment of the invention; and

FIG. 21 is a top view of the microfluidic device packaged in an embodiment of the invention.

DETAILED DESCRIPTION OF THE INVENTION

The structures involved in the invention or the technical terms used are further explained below. Unless otherwise specified, they shall be understood and explained according to the general terms commonly used in the art.

Testing

Testing means to assay or detect presence or absence of a substance or material, including but not limited to, a chemical substance, an organic compound, an inorganic compound, a metabolite, a drug, a drug metabolite, an organic tissue, a metabolite of an organic tissue, a nucleic acid, a protein or a polymer. In addition, testing means testing an amount of a substance or material. Further, assay also means immunoassay, chemical assay, enzyme assay, and the like.

Device

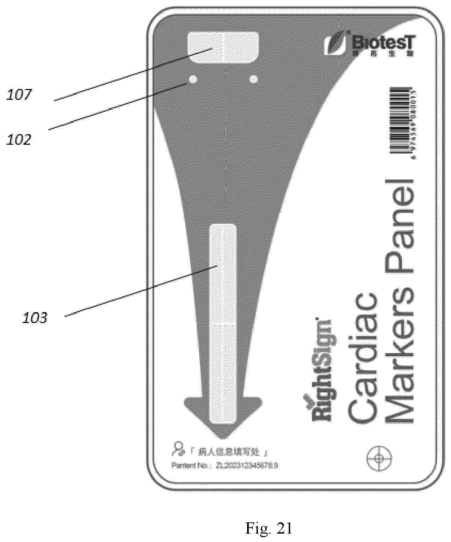

A device includes an apparatus or instrument capable of completing a function. The microfluidic device of the invention may include a disposable chip as described in FIG. 21, through which an analyte in a liquid sample can be tested, and may also include an apparatus for reading a test result, through which the test result of a testing unit in a testing area 114 can be read through a window 103. Therefore, the microfluidic device may also be a microfluidic chip, a fluid test card, a fluid testing element, etc.

Sample

The specimens detected or collected by the test device of the invention include biological liquids (for example, case liquids or clinical specimens), including liquid specimens or liquid samples, or fluid samples or fluid specimens. Here, the specimens and the samples are interchangeable. These samples may be derived from solid or semisolid samples, including feces, biological tissues and food samples. The solid or semisolid specimens may be converted to liquid specimens by any appropriate methods, such as mixing, mashing, macerating, incubating, dissolving, or digesting the solid samples by enzymolysis in suitable solutions, such as water, phosphate solutions, or other buffer solutions. "Biological specimens" include animal, plant, and food derived specimens, including, for example, human or animal derived urine, saliva, blood and components thereof, spinal fluid, vaginal secretions, sperm, feces, sweat, secretions, tissues, organs, tumors, cultures of tissues and organs, cell cultures, and media. Preferably, the biological specimen is urine; and preferably, the biological specimen is saliva. The food specimens include food-processed materials, final products, meat, cheese, wine, milk, and drinking water. The plant specimens include specimens derived from any plants, plant tissues, plant cell cultures, and media. "Environmental specimens" include specimens derived from the environment (for example, liquid specimens from lakes or other bodies of water, sewage specimens, soil specimens, groundwater, seawater, and waste liquid specimens). The environmental specimens may further include sewage or other wastewater.

An appropriate device according to the invention can be used for testing any analyte. Preferably, the test device of the invention is used to detect small drug molecules in saliva and urine or viruses in blood samples, such as human immunodeficiency virus (HIV) and coronavirus, or any other substances of interest. In a preferred implementation, the liquid sample of the invention is a blood sample, including whole blood, serum or plasma.

Liquid and liquid sample

The liquid of the invention usually refers to a liquid sample, but when "liquid" is used alone, it may only indicate a state, but does not necessarily mean that it is a liquid sample. It may also be any other type of liquid, such as a buffer solution, a cleaning solution and an aqueous solution that changes the test performance. For example, when explaining the flow of the liquid under the action of the channel, it only indicates the properties of the channel, in which the liquid sample or other liquids may flow. For another example, the channel has capillary force, so that the liquid can flow by the capillary force. The liquid here is only used to indicate the properties of the channel, and does not necessarily mean that there is a liquid or liquid sample in the channel. For another example, the liquid can flow in the channel under the action of the pump, which only means that the pump has this action but does not necessarily mean that the channel has a liquid or liquid sample therein. Alternatively, the liquid can flow by the capillary force in the channel. The liquid here only indicates the properties of the channel, and the liquid flowing in the channel may be any other liquids, certainly including the liquid sample of the invention, such as blood, urine, sweat or any other samples as defined above.

## Downstream and upstream

Downstream or upstream is divided according to the flow direction of a liquid. Generally, a liquid flows from an upstream area to a downstream area. The downstream area receives a liquid from the upstream area, and a liquid also may flow to the downstream area along the upstream area. Downstream or upstream here is generally divided according to the flow direction of the liquid. For example, on some materials or microfluidic channels that use capillary force to promote liquid flow, the liquid flows by capillary force, for example, the liquid may enter an opening (e.g., an inlet 1074 of the channel) of a dried reagent area 112 from a sample application hole 101. In the case that the liquid enters the microfluidic channel from the opening 1074 of the dried reagent area, before the liquid enters the channel, the channel has capillary force. Once the liquid enters, the liquid is allowed to flow from the dried reagent area 112 (upstream, the opening 1074) to a downstream outlet 1122, for example, a choke area 211 or a mixing area 113, by the capillary force of the microchannel. In some embodiments, a sample channel 1078 is further arranged upstream of the inlet 1074 of the channel, the sample channel is connected to the sample application opening 101, and the sample is added to the opening 101, enters the sample channel 1078 and then flows into the inlet 1074 of the channel, i.e., the inlet of the dried reagent area. Of course, by the capillary force, the liquid may also flow from the mixing area 113 to a downstream testing area 114, and also to a downstream waste liquid area 115. It can be understood that as long as the channel has capillary force and is wetted with the flow of the liquid, the capillary force of the wetted channel will generally disappear. Thus, in some embodiments, an opening 104 at the other end of the microfluidic channel may be connected with a pump, which is configured to extract gas to form subatmospheric or negative pressure in the channel, thereby driving the liquid to flow in the channel. Therefore, if the pump functions to extract gas, then the subatmospheric or negative pressure is formed. If the pump functions to blow gas into the channel, then positive pressure is formed. The positive pressure can overcome the capillary force and make the liquid flow back, so that the liquid can reciprocate in the microfluidic channel. If the subatmospheric or negative pressure is formed, the liquid is allowed to flow from upstream to downstream in the channel by the capillary action in combination with the subatmospheric or negative pressure applied by the pump. Of course, in some embodiments, the liquid can flow from upstream to downstream in a part of the channel only by the capillary force, and in some areas, the liquid can flow under the capillary force of the channel in combination with the action of the pump, which can increase the flow velocity of the liquid. In some embodiments, the channel has little capillary force, and the liquid flows in the channel completely by the action of the pump. The "pump" here is any apparatus that can make the gas in the channel flow to form positive pressure or subatmospheric or negative pressure, such as an electronic pump, a syringe pump, a ceramic pump, etc., which can be used as a specific example of the pump. Of course, under the action of the pump, the liquid can flow from the inlet of the channel to the waste liquid area along the channel, or the liquid can flow from the waste liquid area back to the inlet of the channel or reciprocate in a certain area, for example, reciprocate in the testing area. Therefore, which areas use a pump and which areas only depend on the capillary force can be determined by setting or through specific experimental processes.

## Gas communication or liquid communication

Gas communication or liquid communication means that a liquid or a gas can flow from one place to another. In the flow process, the liquid or the gas may pass through some physical structures that play a guiding role. The "pass through some physical structures" here means that the liquid passes through the surface of these physical structures or the internal space of these structures and flows to another place passively or actively. The passive flow is usually caused by external forces, such as the flow under the capillary action. In the invention, the liquid may flow in the channel from upstream to downstream by the capillary force in the channel or by the action of the pump. The flow here may also be a flow due to self-action (gravity or pressure) of a liquid or a gas, and also may be a passive flow. The communication here does not mean that a liquid or a gas is necessarily present, but only in some cases indicates a connection relationship or state between two objects. If a liquid is present, it can flow from one object to another object. The flow may depend on the capillary force or on the extraction or blowing of gas by the pump. Here, it means a state in which two objects are connected. On the contrary, if there is no liquid communication or gas communication between two objects, and if a liquid exists in or on one object but cannot flow into or on another object, such a state is a non-communication, non-liquid communication or non-gas communication state.

## Analyte

Examples that can use an analyte related to the invention include some small-molecule substances, including drugs (such as drug of abuse). "Drug of Abuse" (DOA) refers to the use of a drug (typically functions to paralyze the nerves) not directed to a medical purpose. Abuse of these drugs will lead to physical and mental damage, dependency, addiction and/or death. Examples of abuse of drug include cocaine; amphetamine (AMP) (e.g., Black Beauty, white amphetamine tablets, dexamphetamine, dexamphetamine tablets, and Beans); methamphetamine (MET) (crank, meth, crystal and speed); barbiturate (BAR) (such as Valium®, Roche Pharmaceuticals, Nutley, and New Jersey); sedatives (i.e., a sleep aid medicine); lysergic acid diethylamine (LSD); inhibitors (downers, goofballs, barbs, blue devils, yellow jackets, and methaqualone); tricyclic antidepressants (TCAs, i.e. imipramine, amitriptyline, and doxepin); dimethylenedioxymethylaniline (MDMA); phencyclidine (PCP); tetrahydrocannabinol (THC, pot, dope, hash, weed, etc.); opiates (i.e., morphine (MOP) or opium, cocaine (COC), heroin, and hydroxydihydrocodeinone); and anxiolytic drugs and sedative-hypnotic drugs. The anxiolytic drugs are mainly used for relieving anxiety, tension, and fear, and stabilizing emotion, and have hypnotic and sedative effects. The anxiolytic drugs include benzodiazepines (BZO), atypical benzodiazepines (BZ), fused dinitrogen NB23C, benzazepines, ligands of BZ receptors, open-ring BZ, diphenylmethane derivatives, piperazine carboxylates, piperidine carboxylates, quinazolinones, thiazine and thiazole derivatives, other heterocycles, imidazole-type sedative/analgesic drugs (e.g., oxycodone (OXY) and methadone (MTD)), propylene glycol derivatives-carbamates, aliphatic compounds, anthracene derivatives, and the like. The test device of the invention may also be used for detecting drugs belonging to a medical use but easy to be taken excessively, such as tricyclic antidepressants (imipramine or analogues) and acetaminophen. These drugs are metabolized into micromolecular substances after being absorbed by human body. These micromolecular substances exist in blood, urine, saliva, sweat and other body fluids or in some body fluids.

For example, the analyte detected by the invention includes but is not limited to analytes in blood, such as tumor markers and myocardial infarction markers in some blood, or creatinine, bilirubin, nitrite and (nonspecific) proteins in urine, hormones (for example, human chorionic gonadotropin, progesterone, follicle-stimulating hormone, etc.), blood, leucocytes, sugar, heavy metals or toxins, bacterial substances (such as proteins or carbohydrates against specific bacteria, for example, *Escherichia coli* 0157:H7, *Staphylococcus, Salmonella, Fusiformis, Camyplobacter genus, L. monocytogenes, Vibrio,* or *Bacillus cereus*) and substances related with physiological features in a urine sample.

Flow of a liquid

The flow of a liquid generally refers to the flow from one place to another. Under normal circumstances, the flow of a liquid in nature mostly depends on the action of gravity to flow from high places to low places. The flow here also depends on external forces, i.e., external gravity, which can become the flow under normal gravity. **In** addition to gravity, the flow of liquid may also overcome gravity to move from a low place to a high place. For example, a liquid can flow from a low place to a high place through extraction, compression or pressure received by it. Alternatively, a liquid against its own gravity in relation to pressure can flow. Of course, the liquid may flow in the microchannel by the capillary force, and under the action of capillary force, the liquid may move from low to high against the gravity. The invention belongs to a microfluidic system, and the flow of the liquid in the channel in the device of the invention basically belongs to a "laminar flow". Therefore, unless otherwise specified in the invention, the so-called flow of the liquid or liquid sample refers to the laminar flow of the liquid. **In** the microfluidic device is in use, the device is laid flat, and the liquid moves along the horizontal direction. Of course, if the device is placed upside down, the liquid may also move upward against the gravity.

Flow velocity of liquid

Microfluidics is a technology to manipulate fluids on a micron scale, which is widely used in the fields of chemistry, biology, and medicine. The flow behavior of liquids in microfluidic systems is significantly different from that on the macro scale. **In** microfluidics, the flow of liquid is mainly laminar flow. The so-called laminar flow means that in the flow process of fluid, each flow layer is parallel to the flow direction or has the same flow direction, and the fluid particles move along a smooth path without obvious mixing or disturbance between adjacent flow layers. Therefore, when microfluidic chips are used for testing, the mixing efficiency is low. Laminar flow main has the following characteristics: smooth streamlines: the fluid particles move along smooth and regular paths, and the streamlines are clear and do not cross; no turbulence: the flow is stable with no vortices or turbulence; low Reynolds number: the laminar flow usually occurs when the Reynolds number is low (Re<2000); and velocity distribution: if in a circular tube, the velocity distribution of the laminar flow is parabolic, where the velocity is the highest at the center and gradually decreases to zero near the tube wall. **In** the invention, some channels are rectangular, the flow velocity of layers in the laminar flow is also the highest at the center and decreases near the edge of the channel. The invention has a relatively large improvement in how to increase the mixing ability of liquid in the laminar flow environment, which will be described in detail later.

Generally, Reynolds number is an important parameter to determine the flow state, which is defined as:

$$Re = \frac{\rho \cdot v \cdot L}{\mu}$$

where $\rho$ is the fluid density; v is the fluid velocity; L is the characteristic length (i.e., tube diameter); and $\mu$ is the fluid viscosity. When the Reynolds number is low (Re<2000), the flow is usually laminar flow. When the Reynolds number is high (Re>4000), the flow is usually turbulence. As can be seen from the above formula, in general, in the testing field, the fluid density and flow viscosity are relatively constant. For example, the density and viscosity of blood samples, urine, whole blood, plasma and serum are relatively stable in a range, and the ratio of density to viscosity is also in a relatively stable range and can sometimes be considered as a constant in some specific samples. For example, for a specific sample, this ratio is in a definite range. Generally, the ratio of density to viscosity of whole blood is approximately in the range of $52.5 \times 10^3$ s/m$^2$ to $350 \times 10^3$ s/m$^2$. Although different whole blood samples have different ratios, but the relative range is fixed. Thus, under the action of a specific sample, the Reynolds number is directly proportional to the size of the tube (in an appropriate range) and the flow velocity. The greater the flow velocity, the greater the Reynolds number, and the greater the diameter, the greater the Reynolds number.

In microfluidics, the flow of liquid is driven, for example, mainly by pressure drive, electroosmotic flow, capillary force and thermal effect. The pressure drive is to push the fluid by external pressure (such as a syringe pump), and is suitable for continuous flow. The capillary force is also a method to drive liquid, which does not need external driving force. In some embodiments, an external pump and capillary force of the microchannel (internal self-driving) are selected to serve as the main driving forces for the flow of liquid, which affects the flow properties of the liquid, for example, the flow speed whether it has mixing characteristics.

The so-called capillary force, also known as capillary effect or capillary force, is an adsorption phenomenon driven by liquid surface tension. Basic principle: the capillary force is generated mainly because of the action of liquid surface tension. Inside a narrow tube or porous object, the liquid can go up along the surface of the object against the gravity until it reaches the maximum height defined by the contact angle of the surface of the object. This is because a surface structure similar to an elastic membrane is formed due to the mutual attraction between liquid molecules. When a liquid comes into contact with a solid surface, the attraction of solid molecules to liquid molecules exceeds the attraction between liquid molecules, such that the liquid is adsorbed and spread out on the solid surface. Three-phase interface and liquid meniscus: the generation of capillary force is closely related to the meniscus on the three-phase interface (liquid, solid and gas). When the liquid rises or falls in a narrow tube or porous material, the liquid surface becomes curved. The presence of the curved liquid surface is the key to generating capillary force. The capillary force always points to the concave surface of the liquid surface, because the concave liquid surface exerts tension on the liquid below while the convex liquid surface exerts pressure on the liquid below. Factors affecting capillary force: The magnitude of capillary force is affected by many factors, including: surface tension of liquid: the greater the surface tension of the liquid, the greater the capillary force; radius of capillary tube (circular tube): the smaller the radius or diameter of the capillary tube, the greater the curvature of the liquid surface in the capillary tube, and thus the greater the capillary force generated; and contact angle between liquid and solid: the contact angle reflects the wetting degree of a liquid to a solid surface, where the smaller the contact angle, the better the wettability of the liquid to the solid surface, and the greater the capillary force. Calculating formula: the formula for calculating the capillary force is:

$$F_{\text{capillary}} = \frac{2\gamma \cos\theta}{r}$$

where F is the capillary force, $\gamma$ is the surface tension of the liquid, r is the radius of the capillary tube, and $\theta$ is the contact angle (the contact angle between the liquid and the channel wall). This formula indicates that the capillary force is directly proportional to the surface tension of the liquid and inversely proportional to the radius of the capillary tube. That is, the greater the radius of the capillary tube, the smaller the capillary force, and the smaller the radius of the capillary tube, the greater the capillary force. Surface tension: there is intermolecular force between liquid molecules close to the molecular surface, that is, surface tension. This force makes liquid molecules gather on the surface to form a relatively stable film. The interaction between liquid molecules inside and outside the capillary tube and the capillary wall makes the liquid form a curved contact angle on the inner wall of the capillary tube.

Generally, in a microfluidic system, the flow velocity of laminar flow is usually calculated based on Hagen-Poiseuille equation:

$$v = \frac{\Delta P \cdot r^2}{8\mu L}$$

where v is the average flow velocity, $\Delta P$ is the pressure difference (generated by capillary force), $r$ is the radius of the channel, $\mu$ is the liquid viscosity, and L is the length of the channel. The greater the pressure difference, the greater the flow velocity.

When the capillary force drives the liquid to flow, the formula of the pressure difference is:

$$\Delta P = \frac{2\gamma \cos\theta}{r}$$

The pressure difference formula is put into the flow velocity formula to obtain the following formula:

$$v = \frac{2\gamma \cos\theta \cdot r^2}{8\mu L \cdot r} = \frac{\gamma \cos\theta \cdot r}{4\mu L}$$

Although some formulae use the radius as the parameter, the radius can actually represent the cross-sectional area of the channel. If the channel is circular, it is related to the radius. If the channel is square or rectangular, it is related to the side length or the cross-sectional area. If the channel is irregular, the capillary force and the pressure difference are actually both related to the cross-sectional area. The smaller the cross-sectional area, the greater the capillary force, the greater the pressure difference, and the greater the flow velocity. Conversely, the greater the cross-sectional area, the smaller the capillary force, the smaller the pressure difference, and the lower the flow velocity.

Although the capillary force and the pressure difference are related to the size of the channel, the flow velocity of the liquid in the channel has a very complicated relationship. The pressure difference $\Delta P$ generated by the capillary force is inversely proportional to the size of the channel, but the flow velocity depends on not only the pressure difference, but also the geometric dimension (radius $r$ or side length $a$) of the channel. The pressure difference $\Delta P$ generated by the capillary force increases as the size of the channel decreases, which tends to increase the flow velocity. However, a significant decrease in the size of the channel will increase the flow resistance, thus inhibiting the increase of the flow velocity. Therefore, when the size of the channel is within a reasonable range, the flow velocity increases with the decrease of the size of the channel and decreases with the increase of the size.

As can be seen from the above formulae, the relationship between capillary force and laminar flow in a microfluidic system is as follows: the pressure difference generated by capillary force drives the fluid to form the flow velocity of laminar flow in the microchannel, and the flow velocity is determined by the capillary force, the pressure difference and the size of the channel (the radius of the circular channel). For the size of the channel within an appropriate range (micron size, for example, about 1-100 microns), as the size decreases, the capillary force increases, the pressure difference increases, and the flow velocity increases. However, if the size is too small (nano size, for example, less than 1 micron), as the size decreases, although the capillary force increases and the flow velocity increases, the resistance also increases sharply, which leads to a decrease of the flow velocity.

Therefore, within the micron size range, the smaller the size, the greater the capillary force, which can promote the flow of the liquid and also increase the flow velocity of the liquid. Within this range, although the resistance of the fluid exists, it does not reach a very high level. Generally, the flow velocity is directly proportional to the capillary force and inversely proportional to the size of the channel. If the size of the channel is excessively large, for example, greater than 100 microns, the capillary force is small, and it is expected to drive the liquid to flow by external driving force, for example, the action of a pump.

Therefore, in the invention, the size of the channel (typically referring to the radius of a circular channel or the side length of a regular square or rectangular channel) is set within the micron size range, for example, 1-100 microns. Certain parts of the channel may also have a size of greater than 100 microns. The size here may be the radius of a circular channel or the perimeter of a regular square or rectangular channel. When area is used as the unit for size, the cross-sectional area under micron size is 1 square micron to 10,000 square microns. As the cross-sectional area decreases, the capillary force increases, and the flow velocity increases. Conversely, as the cross-sectional area increases, the capillary force decreases, and the flow velocity decreases. Within the range of under nano size, for example, less than 1 square micron, as the capillary force increases, the resistance increases sharply, so the flow velocity decreases.

Relationship between the width or radius of the channel and the size of the channel: Generally, the capillary action is related to the size of the channel. The size of the channel affects the capillary force, and the capillary force affects the flow velocity of the liquid in some cases. According to the above introduction, in the micron size range, the capillary force decreases with the increase of the size of the channel, and increases with the decrease of the size of the channel. Here, the size includes the radius (when the channel is circular), cross-sectional area, or width and height of the channel. In the invention, when the size is used as the term, it includes the above range of size. When the cross section of the channel is rectangular, the width and height directly affect the cross-sectional area. When the height is substantially the same, the width determines the magnitude of the capillary force. When the width is substantially the same, the height determines the capillary force. When the cross section is irregular, the cross-sectional area affects the magnitude of the capillary force. The greater the cross-sectional area, the smaller the capillary force, and the smaller the cross-sectional area, the greater the capillary force. Therefore, in the invention, various definitions have different descriptions in specific embodiments, and these different descriptions or explanations al belong to a specific implementation of the invention and do not limit the scope of the invention in any form. For the scope, reference may be made to the claims.

In specific embodiments of the invention, the size of the channel indicates the cross-sectional area of the channel unless otherwise specified. In some cases, when describing the width of the channel, the height is generally constant. Of course, in some cases, both the width and the height change, which can indicate the change of cross-sectional area. Therefore, if the channel of the invention is circular in design, microchannels can be designed with a radius of 1-100 microns, and of course, the microchannels can also be designed with cross-sectional area. The microchannels with a range of 1 square micron to 10000 square microns are all specific embodiments of the invention, and they all have capillary force.

According to the above principle, in some embodiments of the invention, the choke area 211, the dried reagent area 112 connected to the choke area 211, the buffer area between the choke area 211 and the mixing area 113, and the buffer area between the mixing area 113 and the testing area 114 change in size, so that the capillary force of the channel changes and the flow velocity of the liquid changes. This includes the change of the above areas in size, so that the flow velocity of the liquid in these areas changes (which will be described in detail later). In an implementation of the invention, the capillary force is changed by changing the radius, cross-sectional area or width (the height is constant) of the channel, so that the capillary force decreases in some areas of the channel and increases in some areas. In this case, the material of the channel itself is basically the same, but the cross-sectional area of the channel can change, regardless the shape of the tube. The cross section of the tube may be of any shape, for example, a circle, square or rectangle, ellipse, rhombus and other irregular shapes. In general cases, the greater the cross-sectional area of the tube, the smaller the capillary force, the lower the flow velocity. Conversely, the smaller the cross-sectional area of the tube, the greater the capillary force, the greater the flow velocity. In some embodiments, the change of the area may be the increase of the width. When the width increases, the height is generally constant, which can also change the capillary force of the channel. As the width increases, the capillary force decreases, and the flow velocity of the liquid decreases. As the width decreases, the capillary force increases, and the flow velocity increases. If the height of the channel changes, the width may not change, which can also change the capillary force of the channel. In some embodiments, both the height and the width change, which can also change the capillary action of the channel. Therefore, when the flow of the liquid is driven by the capillary action of the channel, the capillary force can be increased or decreased by changing the size of the channel.

In some embodiments, the channel formed by the top cover 105 and the bottom cover 106 is formed by providing a groove (microgroove) in the lower cover and then covering the microgroove with the top cover. The size of the channel depends on the depth or width of the channel. The channel may also be formed by making an inner surface 1051 of the top cover combined with an upper surface 1061 of the bottom cover face to face by laser engraving. When both upper and lower surfaces are smooth, the magnitude of the cross-sectional area of the channel may depend on the depth or width of the channel. The magnitude of the cross-sectional area of the channel is closely related to capillary force. Although the size of the channel is inversely proportional to the capillary force, in a laminar flow system, the flow velocity is inversely proportional to the size of the channel within a certain range (micron size). The smaller the size of the channel, the greater the flow velocity, and the greater the size, the lower the flow velocity. In microfluidic testing, a very important function is to control the flow velocity of the liquid sample in the channel to treat the liquid sample and achieve the purpose of the test.

Of course, the inner surface of the channel may be hydrophilically or hydrophobically treated to change the contact angle between the liquid and the solid surface. The smaller the contact angle, the greater the capillary force, and the greater the flow velocity. Conversely, the greater the contact angle, the smaller the capillary force, and the lower the flow velocity. Different inner surfaces have different hydrophobic properties and different capillary forces, which affects the flow velocity of the liquid. For example, if the inner surface is treated with a hydrophilic reagent, the capillary force increases, and the flow velocity increases. If the inner surface is treated with a hydrophobic reagent, the capillary force decreases, and the flow velocity decreases. Changing the properties of the liquid sample such as urine and a blood sample, or filtering the blood sample, which also changes the properties of the liquid, is generally not commonly used, but it is also an implementation of the invention. In general cases, the microfluidic device arranged can adapt to different liquid samples. By setting the internal structure with microchannels and changing the properties of the inner surface, the microfluidic device can be used for testing trace amounts of liquids, especially for controlling the flow properties of the liquid sample in microchannels.

Therefore, one method is to treat the inner surface of some areas of the channel into a hydrophobic surface. The inner surface of the choke area of the channel can be made hydrophobic using hydrophobic compounds, such as aliphatic and/or aromatic compounds, various inks and polymers, etc. The compounds are generally dissolved in organic solvents or mixtures of aqueous and organic solvents. U.S. Pat. No. 7,824,611 (which is incorporated by reference herein) discloses suitable techniques (such as ink jet printing, paint spraying, silk screening, sketching, embossing and the like), which permit the application of hydrophobic areas on or within surfaces. U.S. Pat. No. 7,824,611 discloses a series of techniques, which can be used for making the hydrophobic surface. For hydrophilic surfaces, hydrophobic zones can be created by application of organic solvents that destroy the plasma treatment or denature the proteins, to recreate a native hydrophobic plastic surface or to create a hydrophobic surface by the denatured proteins, or by local heating of the surface using focused laser beams to destroy the hydrophilic nature of the surface. Alternatively, one can mask hydrophobic areas before creating a hydrophilic area by any of the foregoing methods. The areas can be masked by objects such as a template or can be masked by materials that are applied to the surface and then are subsequently removed.

In an embodiment, the hydrophobic surface is created by starting from the hydrophobic surface, such as are found on native plastics and elastomers (polyethylene, polypropylene, polystyrene, polyacrylates, silicon elastomers and the like). In an embodiment, hydrophobic particles may be placed on a surface. Such particles include latex particles, such as polystyrene latexes with diameters of between about 0.01 μm and 10 μm or hydrophobic polymers, such as polypropylene, polyethylene, polyesters and the like. In another embodiment, the hydrophobic surface can be created by application of a hydrophobic chemical, such as an ink or a long chain fatty acid, or a hydrophobic decal to the desired zone. The hydrophobic chemical or decal is generally not soluble or is poorly soluble in the reaction mixture. In yet another preferred embodiment, the hydrophobic surface can also be formed by changing a hydrophilic surface to a hydrophobic surface. For example, hydrophobic surfaces made hydrophilic by plasma treatment can be converted back to a hydrophobic surface by the application of solvents, ultraviolet light or heat and the like. These treatments can act to change the molecular structure of the hydrophilic, plasma modified surface back to a hydrophobic form.

As discussed above, hydrophobic compounds, such as aliphatic and/or aromatic compounds and various inks and polymers and the like can be used for the creation of hydrophobic areas in accordance with the invention. The compounds are generally dissolved in organic solvents or mixtures of aqueous and organic solvents. Those skilled in the art will recognize that a variety of techniques known in the art (such as ink jet printing, spraying, silk screening, drawing, embossing and the like) are techniques that permit the application of hydrophobic zones on or within surfaces.

Channel

The channel here is similar to a tube or carrier for a liquid to flow, which has an inlet and an outlet and is closed or partially closed in other places. For example, the channel has only one inlet and one outlet, and other places are not in communication with outside atmosphere. Of course, some places may not be closed. In some embodiments, the channel generally includes an inlet 1074 and an outlet 104, and between the outlet 1074 and the inlet 104 is the channel of the invention. Different areas are distributed in the channel. Some of the areas contain reagents, and some are structures for modifying flow properties of the liquid in the channel or contain some chemical substances for surface treatment in the channel to change the flow performance of the liquid in the channel, or the structures arranged in the channel and the substances for surface treatment in the channel function together to modify the flow performance of the liquid. In some embodiments, the channel is circular as a whole, and of course, the cross section of the channel may of any shape, such as ellipse, rectangle, circle, rhombus or other irregular shapes. In the invention, different areas of the channel have different cross-sectional areas and different shapes. For example, in the dried reagent area, the channel has different cross-sectional areas at different positions, but is in the shape of an isosceles trapezoid as a whole. The cross section of the choke area is in a curve-containing shape. The surfaces of the top cover 105 and the bottom cover 106 are straight, but the opposite surfaces of the channel may contain very short curved surfaces. Of course, when the height of the channel is constant, the width of the channel is related to the capillary force, and when the height is constant, the depth of the channel is also related to the capillary force. When the top cover and the bottom plate are combined, the outlet 104 faces an outlet area 110 of the channel on the bottom plate, thereby forming the outlet 104 of the channel. The outlet may be or not be connected to a pump.

The flow properties of the liquid here may include the flow velocity of the liquid, the flow path of the liquid and the change of microstate in the flow process, such as turbulence, vortices, mutual mixing or flow gathering, and also include driving force that the flow of liquid depends on, for example, whether the flow of liquid depends on capillary force or non-capillary force (external pump) of the microchannels, or completely or partially depends on the capillary force, and the repetition or simultaneous change of two or more of the above different properties.

The flow velocity of the liquid here is the distance of the flow path of the liquid per unit time. The flow path of the liquid here means the route, i.e., moistened area, on the surface of the object through which the liquid flows, which may include the change in the flow direction of the liquid, including the change from a linear motion into a curved motion, or the change from upstream into downstream to downstream to upstream, or the change from a curved motion into a linear motion, or the alternation of a curved motion and a linear motion. In the invention, the liquid generally enters from the inlet 1074 of the channel, and then flows out of the outlet, or flows toward the outlet 104 in a laminar flow manner, for example, to the waste liquid area 115 so as to be stored but not to flow out through the outlet 104. In the flow process, the flow properties of the liquid are different in different areas. For example, when the liquid flows through the dried reagent area 112, the flow velocity gradually increases, and the path is a continuous flow from a larger area to a smaller area (as the size decreases, the capillary force increases, and the flow velocity increases). For another example, the mixing area 113 is arranged to allow the liquid to flow in a non-laminar flow manner such that the sample can be mixed. In this way, while the liquid flows along different flow paths or flows generally in a laminar flow manner forward, the flow direction and path of the liquid change constantly. The liquid is divided and gathered together, or divided and gathered repetitively, or undergoes backflow and vortices when gathered, or the flow path changes from a straight line into a curve (the laminar flow direction changes continuously). The curved flow path in the channel makes the liquid flow forward in a curved manner, for example, in a serpentine, zigzag or Y shape. Moreover, the flow path of the liquid may also include scattering and convergence of the liquid in part of areas in the channel. In addition, the channel here may be circular in cross-section in the usual sense, and of course, may be a channel of any other shape. Any place where a liquid can flow from the inlet to the outlet is a specific implementation of the channel defined in the invention. In some embodiments, the change in the flow properties of the liquid is caused by the arrangement of the microstructures in the channel or the treatment by different chemical reagents on the surface in the channel, or external force (for example, pumping), or another factor or a combination of several factors.

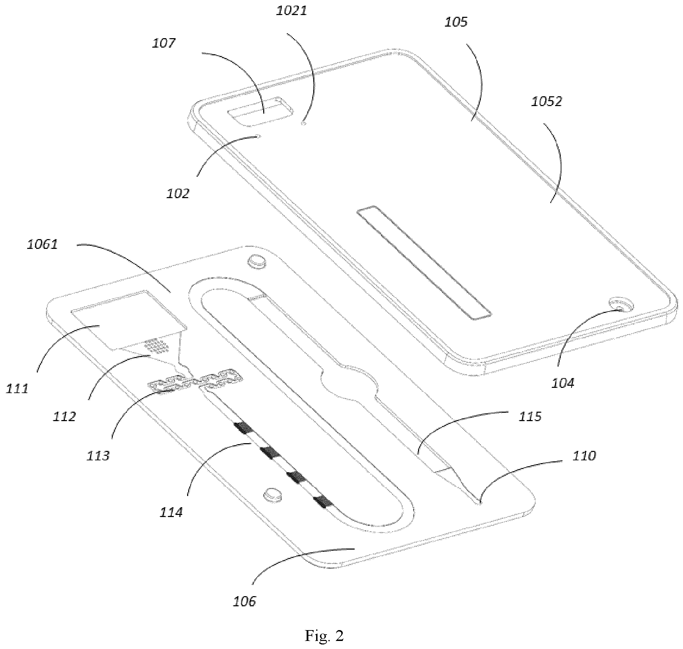

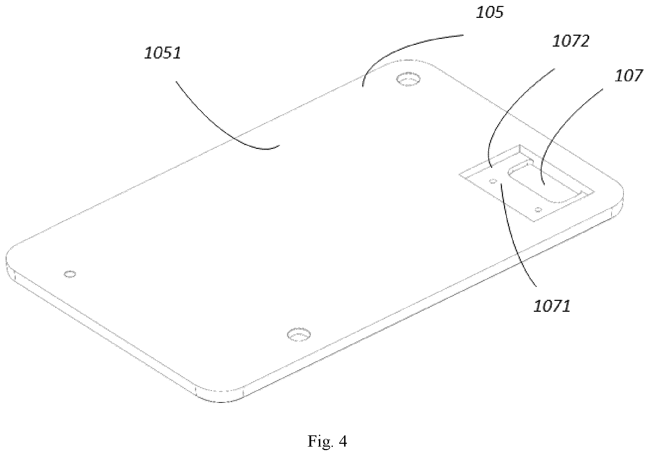

In addition, the channel may be formed in a carrier by an engraving technique, such as a laser technique, or may be formed by forming a groove in a substrate and then covering the groove with a cover plate. In an implementation, the invention provides a cover plate 105 (top cover) and a first base layer 106 (bottom plate or bottom cover). A groove is formed in the base layer, and then the groove is covered with the cover plate 105 to form the channel. The cover plate may be made of a transparent or flexible material, or the top cover 105 and the base layer 106 are both made of a rigid material and formed with microchannels by ultrasonic engraving. The rigid material may be plastic or metal. Of course, the cover plate and the base layer may be made of any material. For example, the base layer is rigid and the cover plate is flexible, or both the cover plate and the base layer are rigid. When both are rigid, the cover plate and the bottom plate may be combined together by a laser technique, and especially the cover plate and the groove may be combined together to form a relatively sealed channel, so that the gas or liquid is allowed to enter or exit only through the inlet and the outlet. Of course, it is best to avoid the risk of leakage when the liquid flows in the channel. Therefore, the channel is generally sealed except the inlet and the outlet, which can avoid leakage of the liquid. In some embodiments, a plurality of channels share one inlet and one outlet. The plurality of channels are formed between the outlet 104 and the inlet 1074, each channel can complete the test of part of analytes, and the liquid sample after the test is finally gathered to a waste liquid area. Alternatively, the liquid sample enters through the inlet, and then the liquid sample is divided into multiple parts which enter different channels to complete the test of different analytes. For example, there may be 2 or more channels, each channel may test one or more analytes, and the testing areas for testing the analytes may be arranged at intervals from upstream to downstream. As shown in FIG. 2, 4 testing areas are arranged in the testing area 114, and each testing area may correspond to one analyte. Of course, different test criteria may be set for one analyte, thus completing a semi-quantitative test.

Components of the microfluidic device, such as the top cover 105 and the base layer 106, may be made of copolymers, blended fibers, laminates, metal foils, evaporated metal films or metals. Optionally, the components of the microfluidic device may be prepared from copolymers, blended fibers, laminates, metal foils, evaporated metal films or metals to deposit one of the following materials: polyolefins, polyesters, styrene-containing polymers, polycarbonates, acrylic polymers, chlorine-containing polymers, acetal homopolymers and copolymers, cellulose and esters thereof, nitrocellulose, fluorine-containing polymers, polyamides, polyimides, polymethyl methacrylate, sulfur-containing polymers, polyurethane, silicone-containing polymers, glass and ceramic materials. By various techniques, including but not limited to gluing, welding, ultrasonics, riveting, etc., the lower and upper base layers 105, 106 can be fixed to each other, various depressions and recesses are sealed, and capillary cavities and the channel are formed.

The channel here may include microchannels (having capillary force) or may also include the sample application channel, also referred to as the sample channel (which may or may not have capillary force) upstream of the microchannels, or the whole of the sample application channel and the microchannels in communication with each other may also be called the "channels" of the invention. The channel here also includes one or more of the dried reagent area, the mixing area, the testing area and the waste liquid area, and additional areas or buffer areas for changing the flow properties of the liquid between the areas. Of course, this channel may also include the sample application channel.

Sample application area

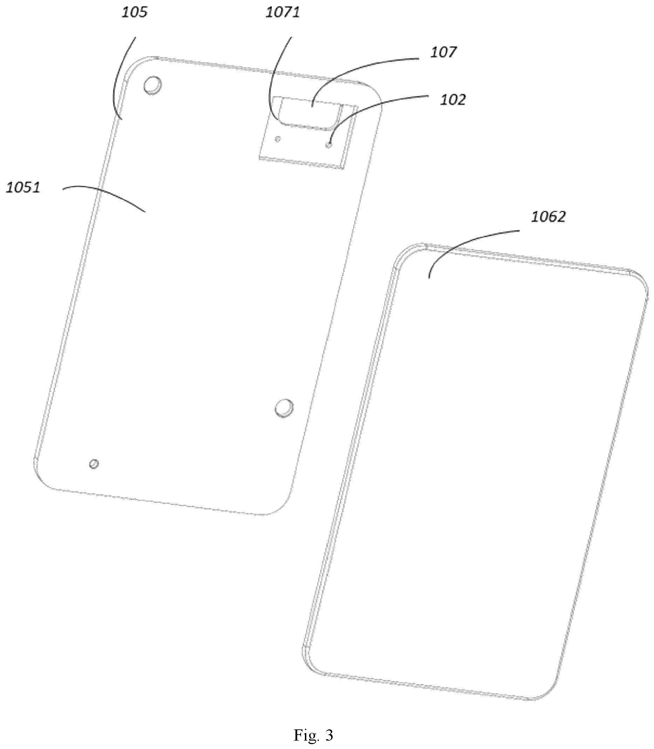

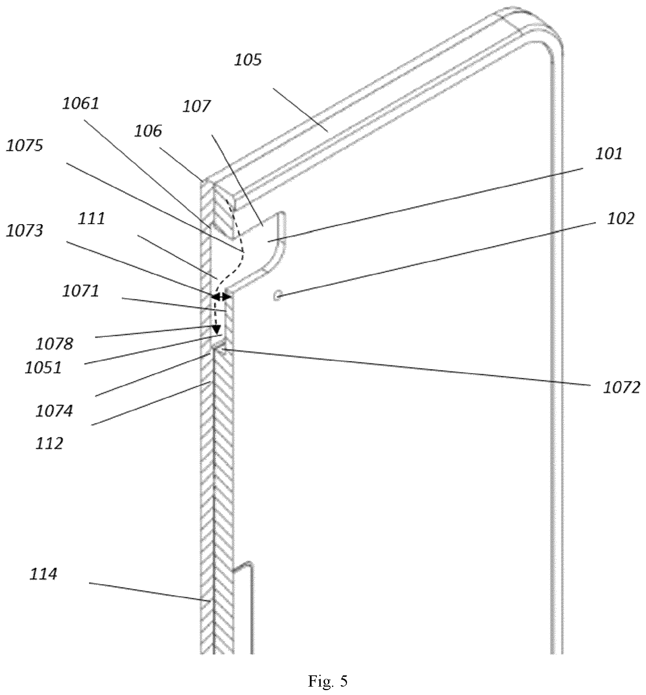

In some embodiments, the invention further includes a sample application area, which is the area where the liquid sample is applied. Generally, the sample application area is provided with one opening through which the sample is added to the microfluidic device such that the liquid sample flows into the microchannels through the inlet 1074 of the channel. In some embodiments, the sample application area includes a sample application opening and a sample application channel 1078. The sample application channel 1078 exists between the sample application opening 107 and the microchannel inlet 1074. In some embodiments, a sample application channel is arranged upstream of the inlet 1074. The sample application channel is formed or assembled by part of areas of the top cover 105 and the base layer 106. For example, as shown in FIG. 2 to FIG. 6, a recessed area 111 is formed upstream of the dried reagent area 112 of the base layer 106, and then the top cover 105 is provided with an opening 107. When the top cover and the bottom cover are combined, the opening corresponds to the recessed area 111 but is located upstream of the dried reagent area, thereby forming a sample application hole 101. The liquid sample is applied through the opening 107 (for example, as shown by the arrow 1075 in FIG. 5), and the liquid sample flows to the sample application channel 1078 and enters the channel through the inlet 1047. In some embodiments, the opening 107 of the top cover 105 is located at one end of the top cover, and the lower area 1071 arranged at the opening 107 is recessed as a whole (the opening 107 is also located in the recessed area), where the depth of the recess is determined by the height of the recessed area 1072. In other words, the sample application opening 107 of the top cover is recessed as a whole, which is thinner than the rest part of the top cover and is recessed by a depth of about 1 micron to 1 millimeter. When the top cover and the bottom cover are combined, a height 1073 is formed between the recessed part on the top cover 105 and the recessed area 111 of the base layer. The height may be 1 millimeter or micron, or a depth of 1 micron or 100 microns or 1-2 millimeters (as shown in FIG. 5). This depth forms a narrow sample application channel 1078. The depth of the sample application channel is formed by the recessed part of the top cover and the recessed area 111 of the bottom plate, and the sample application opening 107 is located upstream of the sample application channel 1078. When the liquid is applied through the opening 107, the liquid generally needs to flow through the sample application channel 1078 and then enter the microchannels through the microchannel inlet 1074. The above is only a specific implementation, and the height or width of the sample application channel may also be formed by providing a recess on one surface of the top cover or the bottom cover. Generally, the height of the microchannels may be approximately micron-sized, for example, 4 to 5 microns, 40 to 50 microns or 40 to 100 microns, or the cross-sectional area of the microchannels is 1 square micron to 10000 square microns, and the height of the sample application channel 1078 may be micron or millimeter or centimeter-sized. In other words, the sample application channel may or may not have capillary force itself. When the sample application channel has capillary force, the sample application channel 1078 (also referred to as the sample channel) and the capillary inlet 1014 of the microchannels have the same width but different heights, which leads to different capillary forces. In some embodiments, generally, the sample application channel 1078 has smaller capillary force, and the microchannel inlet has larger capillary force, so that the added liquid sample can enter the microchannels smoothly and slowly. The microchannels with larger capillary force can drive the liquid of the sample application channel to enter the microchannels. Similarly, if the sample application channel has small or no capillary force, as the liquid sample is added, the liquid enters the sample application channel by the pressure of the liquid itself, and the liquid is driven into the microchannels by the capillary force at the microchannel inlet 1017, but the flow velocity may be lower.

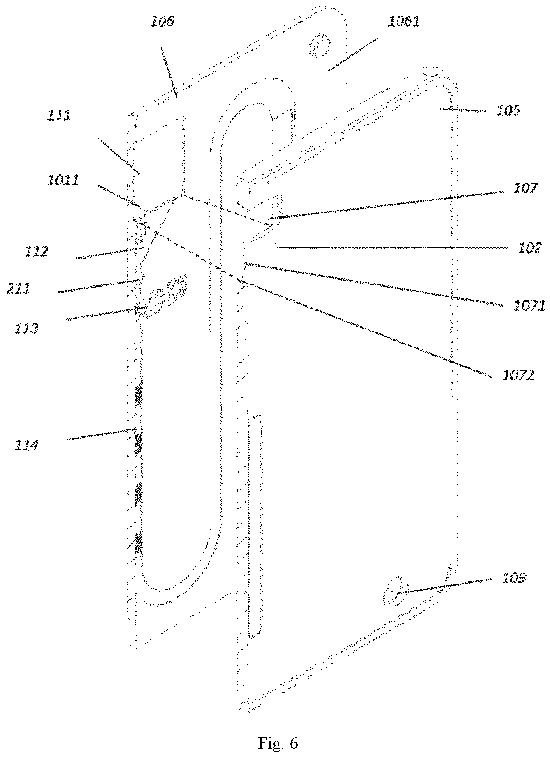

In this way, when the liquid sample is added to the sample application area through the opening 107, the area is filled with the liquid sample, for example, the sample application channel 1078 is filled first, and then, all the sample can enter the microchannels. Therefore, the sample application channel 1078 is used as a transition channel, so that the sample is not directly applied to the inlet 1074 of the channel, which avoids the difficulty in directly applying the sample to the inlet 1074 of the channel. This is mainly because the microchannel inlet 1074 is micron-sized and requires a more precise tool to apply the sample to the channel, and it is not always possible to apply the sample into the microchannels. By arranging the sample application channel 1078 upstream of the microchannel inlet 1074 which has a larger thickness and is in communication with the sample application opening 107, it can basically ensure that the liquid enters the device and fills the whole sample application area. In some embodiments, as shown in FIG. 6, if the microchannel inlet 1074 has a larger width, for example, an edge 1012 of the recess 1071 of the top cover 105 is equal to an edge 1011 of the recessed area 111 of the base layer 106, when the top cover and the base layer are combined, a gap is directly formed, which is the inlet 1074 of the microchannels. The width of the inlet 1074 of the channel is equal to or the same as that of the recessed area 111 and the recessed area 1071 of the top cover 106, but the height is almost micron-sized, which leads to a difference in capillary force between the sample application channel and the microchannel inlet. Generally, the capillary force of the microchannels is greater than or much greater than the capillary force of the sample application channel, for example, the difference in height is millimeter-sized. After the sample is applied through the opening 107, the liquid sample contacts or reaches the inlet 1074 through the sample application channel having the same width as the microchannel inlet 1074, so that the inlet 1074 can come into contact with the liquid sample almost at the same time, and the liquid sample can enter the microchannels with almost the same chance, for example, enter the dried reagent area 112 of the microchannels with almost the same chance. Therefore, in some embodiments, the height of the sample application channel 1078 is greater than the height of the inlet 1074 of the microchannels, and the width is equal to or greater than the width of the inlet 1074 of the microchannels. By arranging the sample application channel 1078, the liquid sample can be buffered before entering the microchannel inlet 1074. First, the sample application channel is filled with the liquid sample, so that the liquid can enter the microchannels evenly in no order and more liquid sample can be accommodated. In this way, not only can the liquid sample be used for testing, but there is also an excess of sample to flush the microchannels to reduce the test background (explained later). Of course in an optional implementation, the width of the sample application channel 1078 may be greater than the width of the microchannel inlet 1074, so that the liquid in the sample application channel filled with the liquid sample can enter the microchannels with the same chance. The "microchannels" here are used only relative to the sample application channel to make the names distinct and not confusing, but do not have any other restrictive effects.

When the sample is applied, it is expected that no gas enters the microchannels. If the gas enters the microchannels, bubbles formed by the gas in the microchannels will affect (in general cases, retard) the flow of the liquid and the test result. For example, if the bubbles pass through the testing area, there will be no sample but bubbles contacting the testing area, which will affect the testing performance (accuracy). Therefore, in some embodiments, the sample application channel 1078 between the sample application opening 107 and the microchannel inlet 1074 is provided with vent holes 102, 1021. When the liquid sample flows through or fills the sample application channel, the gas inside is discharged, and at the same time, the discharged gas allows the liquid to fill the sample application channel 1078 faster. If there are no vent holes, since the sample application channel only has a millimeter-sized height and the sample added through the opening 107 closes the opening, the liquid inside will not flow easily. This is because there is gas in the liquid sample before the microchannel inlet 1074, and as the liquid sample flows, the gas closed therein may be pushed into the microchannels, which reduces the flow velocity of the liquid and makes it difficult for the liquid to flow to the microchannel inlet. Therefore, by providing the vent holes in the sample application channel 1078, the excess gas can be discharged, which can reduce the gas entering the microchannels and drive the liquid to flow in the sample application channel at a higher flow velocity and fill the sample application channel. In some embodiments, the vent holes may be directly provided in the top cover 105 and in fluid communication with the sample application channel 1078. In some embodiments, when the sample application channel has a larger width, a plurality of vent holes can be provided, for example, at two ends of the sample application channel, or near the microchannel inlet 1074, or at any position on the sample application channel formed between the sample application opening 107 and the microchannel inlet 1074. The number of the vent holes may be 1 or more, for example, 2, 3 or 4.

In some embodiments, a sample pretreatment layer, for example, a filter layer made of a porous material, may be arranged in the sample application area. The filter layer can pretreat the sample to remove impurities or other interfering components or entrap red blood cells (if the sample is a blood sample). Thus, the pretreatment layer may be provided with some chemical reagents in advance to change the shape, flowing properties or viscosity of the liquid. In some embodiments, the pretreatment layer is arranged in the projection area of the sample application opening 107, so that the added liquid sample flows to the sample application channel after flowing through the pretreatment layer. Of course, the thickness of the pretreatment layer is allowed to the less than the height of the sample application channel, and then the sample application channel is allowed to contain the pretreatment layer, so that the liquid sample can flow into the microchannel inlet 1074 and enter the microchannels only after flowing through the pretreatment layer. The pretreatment layer is an optional solution. In some embodiments, the pretreatment layer may be omitted, so that the liquid can flow into the microchannels faster. After all, the pretreatment layer reduces the velocity of the liquid flowing from the sample application opening 107 into the microchannel inlet 1074 on the whole.

Dried reagent area

In some embodiments, a dried reagent area is arranged in the channel, and dried reagents are arranged in the dried reagent area, or solution reagents are dried to remove water or made into freeze-dried microspheres and are immobilized in the dried reagent area. In a word, the area has dried reagents when testing is carried out. First, the reagents in the area need to come into contact with the liquid sample, such that the dried reagents are dissolved by the liquid sample to leave the dried reagent area, change from a dry solid into a liquid mixed with the liquid sample and flow with the liquid. For example, as shown in FIG. 5 to FIG. 7, the dried reagent area 112 is arranged downstream of the sample application channel 1078, and the liquid sample enters the dried reagent area through the inlet 1074 of the microchannels such that the sample comes into contact with the reagents in the dried reagent area. In the dried reagent area, it is expected that the sample can dissolve, activate and moisten the dried reagents in the area such that the reagents can be moved along with the flow of the liquid sample. In some embodiments, some reagents may be arranged in the dried reagent area in advance by means of processing. The reagents include auxiliary reagents and one or more first receptors which can specifically bind to one or more analytes in the liquid sample. The first receptor(s) can be directly or indirectly coupled with markers, such as fluorescence, dyes, gold particles or latex particles. The dried reagent area with markers may also be called a marking area. In some embodiments, when the liquid flows through the dried reagent area, the main function is to allow the dried reagents in the dried reagent area to be dissolved by the liquid sample and to flow with the liquid sample.

In some embodiments, in order to make the dried reagents be dissolved by the liquid sample and taken to the downstream area as much as possible, it is expected that the liquid sample flows at a lower flow velocity in this area. In other words, it is expected that the liquid sample stays in this area for a longer time and the liquid sample has more time to contact the dried reagents, such that these dried reagents can be substantially hydrated and dissolved completely and the dissolved reagents can flow downstream with the liquid. When the liquid sample has a water content or is relatively viscous, the flow velocity of the liquid sample can also be reduced in this area. For example, the sample may be treated in advance to increase the viscosity, but an intentional increase may affect the subsequent flow properties, so it is generally suggested not to change the original properties of the liquid sample but to maintain the natural viscous properties.

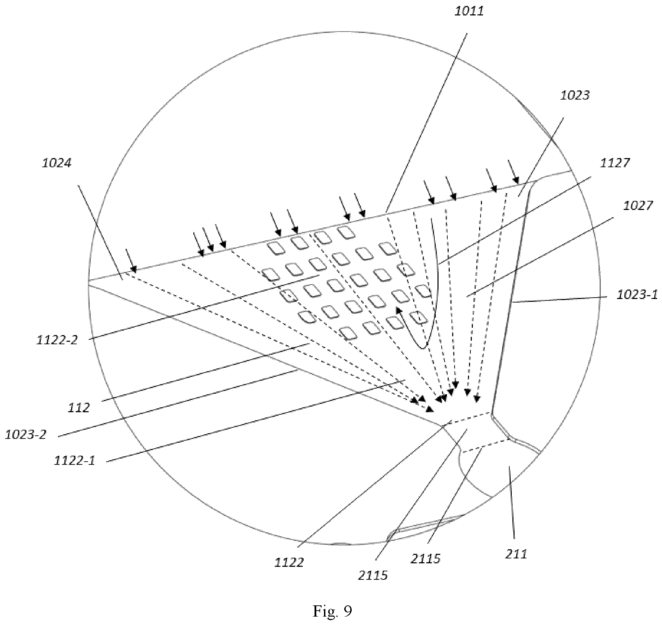

In some embodiments, in order to make the liquid sample stay in the area for a longer time and thoroughly contact and treat the dried reagents in the dried reagent area, a method of changing the flow properties of the liquid may be selected. In some embodiments, the structure of the channel in the dried reagent area is designed. For example, the capillary force of the liquid inlet of the dried reagent area (or the inlet 1074 of the microchannels) is made less than the capillary force of the outlet 1122 of the dried reagent area. A smaller capillary force leads to a lower flow velocity. As shown in FIG. 9, the width of the inlet 1011 of the dried reagent area 112 is greater than the width of the outlet 1122 of the dried reagent area, so that the capillary force gradually increases (in a case where the height of the channel is constant). In addition, the width of the inlet of this area 112 is greater than the width of the outlet 1122, so that the liquid sample flows in through the large inlet and out from the small outlet. With this structural arrangement, in a case where the capillary force is considered, the large inlet has small capillary force, and the liquid flows slowly, so that the liquid can stay in the dried reagent area and contact the dried reagents for a longer time. In addition, the dried reagent area includes a reagent area 1122-2. In order to immobilize the reagents into the dried reagent area, the dried reagent area needs to be roughened to make the surface rough such that the dried reagents can be adsorbed. The rough surface can reduce the flow velocity of the liquid and allow the liquid to stay in this area and contact the dried reagents. Of course, in some embodiments, the downstream of the reagent area 1122-2, especially other areas downstream of the reagent area 1122-2, may be hydrophobically treated. For example, a part 1122-1 upstream of the outlet is treated with a hydrophobic reagent to make this part hydrophobic, so that the flow of the liquid sample flowing in from the inlet 1074 is blocked upstream of the outlet 1122 and the flow velocity is reduced. The hydrophobic treatment actually reduces the capillary force. The area 1122-1 is located between the reagent area 1122-2 and the outlet 1122 and downstream of the reagent area 1122-2, and functions to reduce the capillary force on the liquid and reduce the flow velocity of the liquid. For example, in some embodiments, the reagent area 1122-2 has a rough area 1123 formed by a plurality of protruding columnar structures 1124, 1124 which are distributed in the dried reagent area and used for treating dried reagents. When a reagent-containing solution is added dropwise to the rough surface, the liquid can be fixed. Water can be evaporated by drying, leaving the dried reagents in the reagent area 112-2. Therefore, as shown in FIG. 9, the area (rough area 1123) for fixing dried reagents is close to the inlet 1011 of the dried reagent area and can make the liquid contact the dried reagents in advance so as to accelerate the dissolution of the dried reagents. Moreover, the capillary force at the inlet is smaller, and the flow velocity is lower, so that the liquid sample has more time to contact and dissolve the dried reagents. In another aspect, in some embodiments, with the structural design of the dried reagent area having the larger inlet and the smaller outlet, more liquid enters and less sample exits. That is, a larger volume of sample flows in through the inlet per unit time, and a smaller volume of sample flows out through the outlet per unit time. In this case, although the capillary action of the outlet is greater than the capillary force of the inlet, the width or cross-sectional area of the outlet is smaller, so the liquid entering through the wider inlet 1074 cannot be discharged in time, and the liquid in the dried reagent area stays for a short time or flows back in this area, as shown by arrow 1127 in FIG. 9. In this way, the liquid sample can stay in the dried reagent area for a longer time and thus thoroughly dissolve the dried reagents. Although the capillary action gradually increases, the capillary action on the forward flow of the liquid can be counteracted or partially counteracted by means of the change of the structure. In another aspect, the liquid is allowed to flow only by capillary force, and a design with a larger inlet and a smaller outlet (the dried reagent area is located in part of the microchannels) is used. Although the capillary action gradually increases, this increase is slow. In this way, the liquid can stay for a longer time, and the liquid can also have driving force to flow forward. The "gradual" increase here may be a gradual and smooth increase without steps, which depends on the manner in which walls 1023-1 and 1023-2 of the channel between the outlet 1122 and the inlet 1011 change. This increase may be a linear change, a step-by-step change or any other form of change. Therefore, the dried reagent area is designed with a wider inlet and a narrower outlet, and the area between the outlet and the inlet may be in any shape. This is a special design for blood samples (whole blood, plasma and serum), which is an improved design in structure that can solve the problem that the blood samples are viscous. Of course, it is also applicable to other types of samples. However, on the whole, the dried reagent area has larger capillary force than the channel having the same width as the inlet 1074, and smaller capillary action than the channel having the same width as the outlet 1122, so that the dried reagent area has capillary force and can drive the liquid to flow downstream slowly. In an implementation, the dried reagent area is in the shape of a funnel, a trapezoid or an isosceles trapezoid, each of which is designed with a larger inlet and a smaller outlet. For example, the inlet 1011 formed by a connecting line between two points 1123, 1124 and the outlet 1122 at the bottom form an isosceles trapezoid (here, the height of the channel is constant), the width of which gradually decreases. Through this structural arrangement, the flow velocity of the liquid is reduced in the dried reagent area, so that the liquid can flow slowly, thereby providing enough time for the reagents in the dried reagent area to be dissolved by the liquid sample and move downstream.

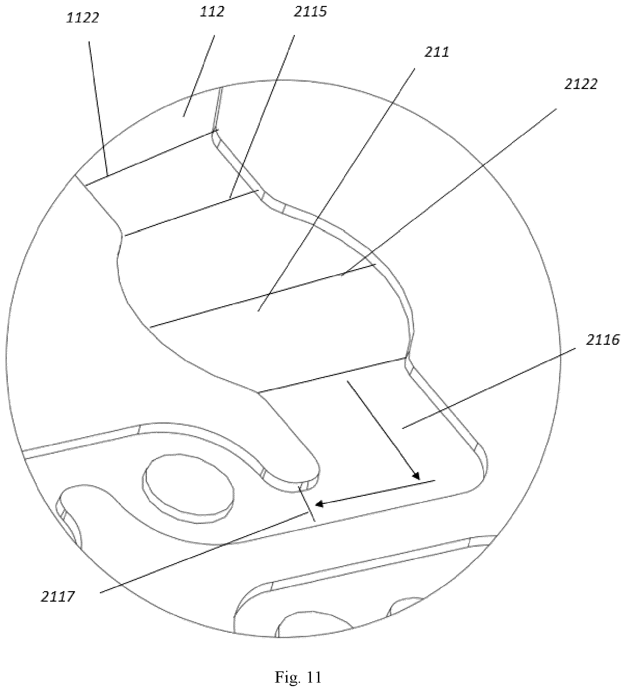

In addition to improving the structure of the dried reagent area itself to reduce the capillary force and change the flow properties of the liquid sample in the dried reagent area, a choke area (similar to a valve) may further be arranged downstream. Therefore, in some embodiments, a choke area 211 may be arranged downstream of the dried reagent area. The choke area can change the flow properties of the liquid, for example, it can reduce or lower the flow velocity of the liquid, and in some cases, can block or stop the liquid from flowing. A method to reduce the flow velocity of the liquid is to change the capillary force. The capillary force can be gradually reduced, and in some cases, there is no capillary force. The capillary force may be changed by structural design or by changing the substances on the surface in the channel. The choke area can reduce the flow velocity of the liquid and allow the liquid to stay in the dried reagent area for a longer time, so that there is more time for the liquid sample to dissolve the dried reagents in the dried reagent area. In some embodiments, capillary force of an outlet area 2115 of the dried reagent area is greater than capillary force of the downstream choke area 211, so that the capillary force of the choke area 211 is decreased or reduced and the flow velocity of the liquid in the choke area is decreased. The decrease in the flow velocity naturally decreases the flow velocity of the liquid in the dried reagent area and reduces the driving force for driving the liquid to flow forward, which is another method for changing the flow velocity of the liquid. A specific method is to make the width of the choke area greater than the width of the outlet 1122 or the outlet transition area 2115 of the dried reagent area (in the case where the height is constant) and increase the radius, so as to reduce the capillary force. As shown in FIG. 11, the width of the outlet 1122 or the transition area 2115 downstream of the outlet 1122 is smaller than the width of the downstream 2122. As the width increases, the capillary force decreases, and the flow velocity of the liquid decreases. For example, the width of the outlet 2112 is 40 microns, and the width 2118 of the choke area 211 is 45, 80 or 100 microns or may be 1 time, 1.5 times or 2 times the width of the outlet. In this case, as the width increases, the capillary force decreases, so that the flow velocity decreases. Alternatively, the cross-sectional area of a certain position of the choke area is greater than the cross-sectional area of the outlet 1122 or a certain position of the outlet transition area 2115, so that the downstream capillary force is reduced, and the flow velocity of the liquid in the choke area is lower (relative to the outlet transition area 2115).

Of course, in other embodiments, the choke area 211 may be treated with a hydrophobic reagent to change the contact angle between the liquid and the solid surface. The contact angle between the liquid and the solid surface may be increased to decrease the capillary force of the choke area. If hydrophobic treatment is performed, the width does not necessarily need to be changed. Of course, the hydrophobic treatment may be performed on the basis of increasing the width. Usually, in order to reduce the cost, the whole channel is injection-molded and assembled from a same material. The capillary force is easily achieved through structural arrangement, but achieving the capillary force by changing the chemical properties of the surface of the channel is not excluded. It can be understood that it is much easier, simpler and cheaper to change the structural arrangement of the channel than to treat the inner surface of the channel with hydrophobic or hydrophilic reagents. Of course, in an optional implementation, the channel in the choke area is flexible and compressible. If the channel in this area is compressed and thus closed (similar to a valve), the closed channel prevents the liquid from passing through, which is also a method to make the liquid sample stay in the dried reagent area for a longer time. In this method, although the capillary force is still present, closing the channel by compressing the channel by external force is still a method to reduce the flow velocity or prevent the liquid from flowing.

In some embodiments, the flow velocity of the liquid is changed by changing the capillary force from the dried reagent area to the choke area. In general, the liquid is allowed to flow forward at a low velocity, and the flow of the liquid sample is achieved only by the capillary force of the channel. The overall purpose is to make the liquid sample contact the dried reagents for a longer time such that the dried reagents contact the liquid sample and are dissolved in the liquid sample. Especially for a blood sample, in a case where the blood sample is not filtered, it takes more time to dissolve and adsorb the dried reagents in the dried reagent area. In some embodiments, the dried reagents include first receptors specifically binding to the analytes, and marking reagents, such as fluorescence, magnetic beads, laser, colloidal gold labels, latex or any other markers. The first receptors may also be antibodies or antibody fragments.

The choke area is also a part of the channel arranged downstream of the dried reagent area. It can be understood that the choke area and the dried reagent area may be directly connected to the channel with a larger width without the transition area 2115. This width is relative to the width of the outlet 1122 of the dried reagent area. According to the principle of capillary action, when the height is constant, the larger the width, the smaller the capillary force, the smaller the driving force on the flow of the liquid, and the lower the flow velocity; and the smaller the width at the outlet 1122, the larger the capillary force. When the liquid flows in the channel from a position with larger capillary force to a position with smaller capillary action, the flow velocity is decreased, so that the driving force for driving the liquid to flow downstream is naturally reduced. In some embodiments, the capillary force of the channel in the whole choke area may be less than the capillary force of the outlet 1122, or the capillary force of one surface may be less than the capillary force of the outlet 1122, which can be freely set by those of ordinary skill in the art on the basis of the invention and can also be specifically modified at will according to the actual situation.

Literally, the choke area functions to reduce the flow velocity of the liquid. However, in some examples of the invention, the choke area may also function to increase the flow velocity of the liquid. The capillary force in this area 211 is allowed to be greater than the capillary force of the outlet 1122 of the dried reagent area or the transition area 2115, thereby increasing the flow velocity of the liquid. In some embodiments, a buffer area is arranged downstream of the choke area. The buffer area can change the flow direction of the liquid flowing therethrough and allow the liquid with a low flow velocity to enter an area with a high flow velocity, such as the mixing area. As shown in FIG. 11, a buffer area 2116 is arranged downstream of the choke area 211. In this area, the forward flow of the liquid changes into a leftward flow and enters the inlet 2117 of the mixing area. Moreover, for the flow properties of the liquid, the buffer area 2116 serving as a transfer area can gather the liquid sample and allow the liquid sample to enter the mixing area smoothly.

Mixing area

In some embodiments, the device of the invention may further include a mixing area, which is also a part of the channel located downstream of the dried reagent area or downstream of the choke area 211 or downstream of the buffer area 2116. The mixing area functions to allow the reagents in the dried reagent area to be thoroughly mixed with and contact the analyte (if any) in the sample, so that the first receptor in the dried reagent area can thoroughly react with the analyte to form a marker-first receptor-analyte complex, which flows to the downstream testing area and is directly or indirectly captured by the second receptor immobilized in the testing area, and thereby, the quantity of the marker is detected to indicate the presence or quantity of the analyte.

In some embodiments, the liquid flows in the mixing area in a non-laminar flow manner to create turbulence and vortices, so as to enhance the mixing effect. Non-laminar flow means that in the flow process of the fluid, the trajectory of fluid particles is irregular, the streamlines are chaotic and the flow state is unstable. The non-laminar flow includes turbulence, transition flow and other complicated flows. The non-laminar flow is beneficial to the mixing of the sample. Chaotic streamlines: the trajectory of fluid particles is irregular, and the streamlines are crossed and chaotic; turbulence: there are eddies, vortices and strong lateral mixing in the flow; high Reynolds number: the non-laminar flow usually occurs when the Reynolds number is high (Re>4000); and energy dissipation: the energy dissipation of the non-laminar flow is large, and the flow resistance of the non-laminar flow is significantly higher than that of the laminar flow. Since the flow resistance of the non-laminar flow is significantly higher than that of the laminar flow, resistance elements or division elements are arranged in the mixing area to divide or separate the channel into a plurality of smaller microchannels so as to increase the capillary force and overcome the flow resistance of the liquid, and moreover, the flow direction changes in parts of the mixing area such that the intensity of turbulence is increased, thereby achieving the purpose of thoroughly mixing. In the mixing area, it is expected that the liquid flows unstably along an irregular flow path, but flows toward the inlet 2117 of the channel into the outlet 113-1 of the mixing area on the whole.

In some embodiments, the mixing area is also a part of the channel, which is generally arranged upstream of the testing area. In some embodiments, when there is no choke area 211 downstream the dried reagent area, the mixing area may be in direct communication with the dried reagent area, and the liquid sample flowing out of the dried reagent area directly flows into the mixing area to be mixed with the reagents and then flows to the downstream testing area. Therefore, the choke area 211, the transition area 2116 from the choke area to the mixing area, and the transition area 119 from the mixing area 113 to the testing area 114 described later are not necessarily required, and they may be partially reserved or totally omitted, all of which belong to a specific implementation of the invention.

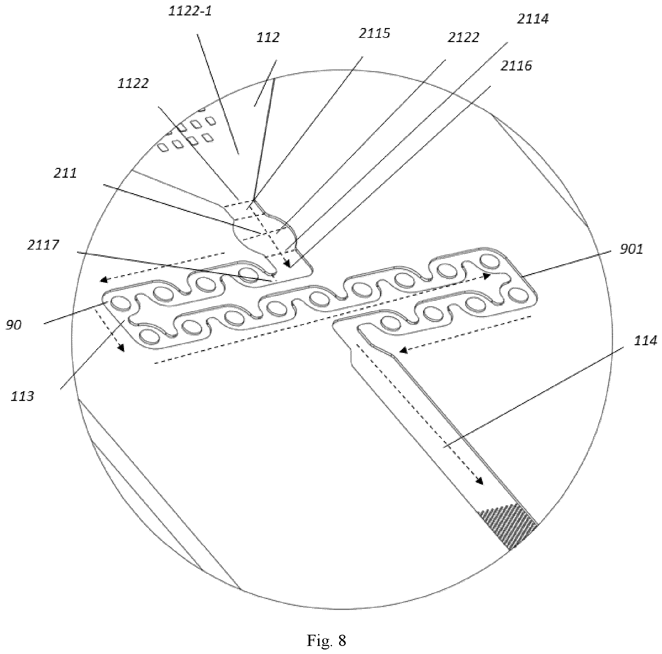

Examples shown in FIG. 8, FIG. 10, FIG. 11, FIG. 12 and FIG. 13 are some specific embodiments of the invention. The mixing area 13 is located at a part of the channel between the dried reagent area 112 and the testing area 114. The mixing area still has the inlet 2117 and the outlet 113-1 of the channel. The inlet of the mixing area is configured to receive the liquid sample (with the reagents of the dried reagent area) from the dried reagent area, and the outlet allows the liquid sample (with the reagents of the dried reagent area) to flow out to the downstream testing area. As shown in FIG. 8, in some embodiments, the inlet 2117 connects the choke area with the mixing area, and the outlet 113-1 connects the mixing area with the testing area. In some embodiments, the buffer area 2116 is arranged between the inlet 2117 of the mixing area and the outlet 2114 of the choke area. The buffer area serving as a transition area allows the liquid from the choke area 211 to flow into the mixing area, and mainly functions to reduce the impact of the liquid sample on the mixing area such that the liquid smoothly enters the mixing area to be mixed after being buffered in this area. In some embodiments, a width of the channel in the buffer area 2116 is greater than a width of the inlet of the mixing area, and the mixing area is required to increase the capillary force to accelerate the flow of the liquid on the whole. Similarly, a transition area 119 is also arranged between the outlet 133-1 of the mixing area and the testing area 114. The transition area 119 is arranged to reduce the velocity of the liquid flowing from the mixing area into the testing area. As can be seen, a width of the transition area 119 is greater than a width of the outlet 113-1, so that the capillary force is reduced, and the flow velocity is lowered. In some embodiments, the channel of the mixing area is generally perpendicular to the testing area and the dried reagent area, so as to save space and reduce the size of the microfluidic chip. In some embodiments, the mixing area is generally present in a folded form, which makes the liquid sample thoroughly contact and react with the dried reagents dissolved in the sample. Especially when the content of the analyte in the liquid sample is very low, a thorough reaction is necessary, which can improve the sensitivity of testing. In a specific implementation of the invention, the mixing area of the invention has only two folds. For example, as shown in FIG. 8, the liquid sample flowing out of the choke area meets the first fold 90 on the left side and then the second fold 901 between the outlet of the mixing area and the testing area. The liquid sample can be thoroughly mixed simply through the two folds, which greatly shortens the flow path of the liquid as compared with multiple folds in the prior art. In addition, in an implementation of the invention, the liquid moves regularly in the mixing area, for example, in a division-gathering-division manner on the whole, so that the liquid sample can thoroughly contact and react with the reagents of the dried reagent area dissolved in the liquid sample.

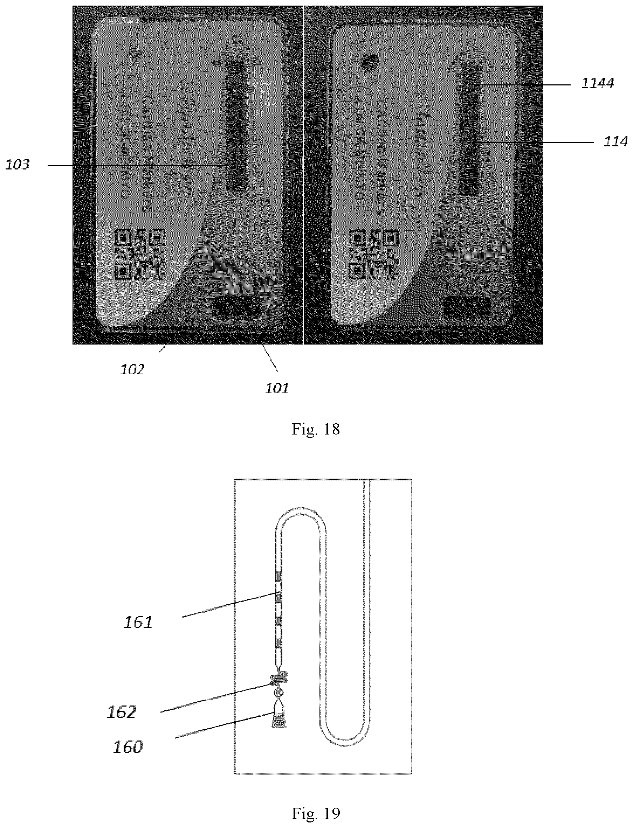

In the traditional design, the mixing area is formed simply by multiple folds of the channel (for example, it is serpentine through multiple bends). This mixing area does not have an ideal mixing effect, and its mixing effect only works at the folds. For some samples having a high viscosity and a low analyte content, it is required to increase the length or the number of folds of the channel in the mixing area and make the liquid flow in a longer channel to enhance the mixing effect. For example, as shown by reference sign 3 in FIG. 2 to FIG. 10 in U.S Pat. No. 11/286,405 (although reference sign 3 in the figures is a testing area in this U.S. patent), a plurality of repetitive folds are arranged to prolong the flow path of the liquid. This design can achieve the purpose of thorough mixing, but there are certain difficulties in design, such as large length, high processing difficulty and unsatisfactory effect. The invention shortens the distance or length of the channel in the mixing area as compared with the traditional design by another method, i.e., arranging some elements for changing the flow path and direction of the liquid in the channel such that the liquid flows forward in the channel in the mixing area in a division-gathering-division manner. In this way, the liquid can be thoroughly mixed, and the substances of the dried reagent area can thoroughly contact the liquid sample so as to achieve the purpose of thorough reaction. Of course, although the structure of the mixing area in this specific implementation of the invention is more advantageous than the traditional method, it does not mean that the traditional structure is excluded as a method. For example, as shown in FIG. 19, the channel in the mixing area may also adopt a reciprocating folding method, for example, the method of the mixing area 16, which is not very effective, but is also an alternative implementation of the invention. For example, the mixing area may be arranged downstream of the choke area in the invention.

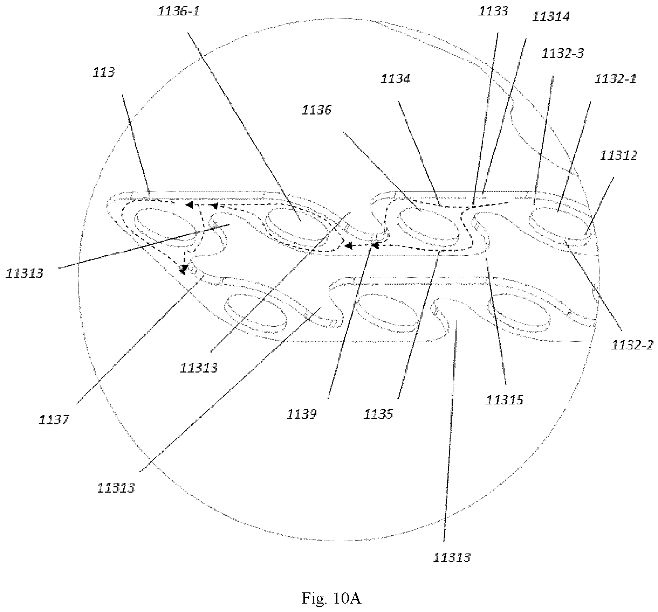

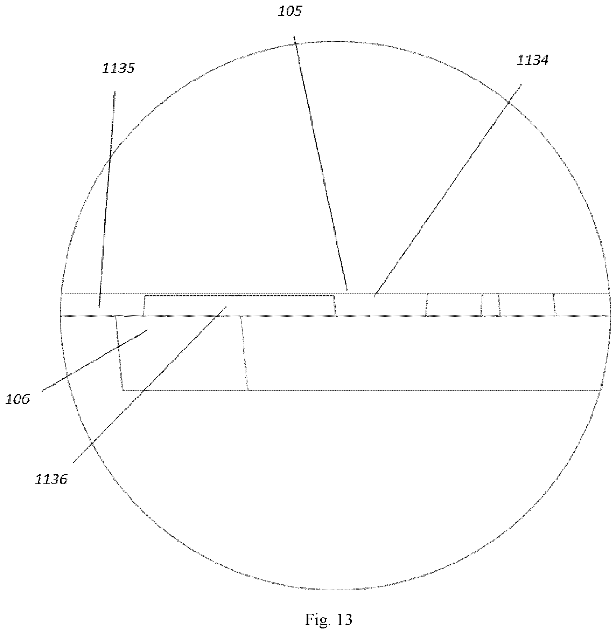

As shown in FIG. 10A and FIG. 13, in some embodiments, a choke element 1136 (the choke element here may also be called a division element, the choke element and the division element are interchangeable) is arranged in the channel in the mixing area. The choke element 1136 is located relatively in the middle of the channel and divides the channel into two channels 1134, 1135 (denoted by the arrows) along the horizontal direction. When the liquid flows forward from the upstream channel 1133, the liquid is divided by the choke element into two flow paths such that the flow direction and path of the liquid are changed, and the direction always changes, which is substantially different from the so-called "laminar flow". Then, the liquid flows to the tail end or downstream position 1139 of the choke element 1136, gathers together, continues flowing forward, is divided and gathers together. When the choke element is circular in design, the liquid flows around the center of circle to form a vortex. In some embodiments, the direction of each divided flow path of the liquid is different. For example, the distance and route of the flow path 1134 around the choke element 1136 are different from those of the flow path 1135 around the choke element 1136. In the case where the distance is different, there is always one flow path arriving at the intersection first, and the other flow path arrives later. The two flow paths of liquid collide with each other at the intersection, such that vortices and turbulence may be formed, thereby achieving thorough contact and thorough mixing between the liquids. Division elements are arranged in the mixing channel. Based on the positions of the division elements, divided channels with different sizes can be formed. Different sizes change the capillary force of the divided channels, thereby changing the flow velocities of the liquids entering the divided channels. Thus, the liquids in different divided channels flow at different flow velocities. For example, the size of the channel of the flow path 1135 is reduced, and the size of the channel of the divided path 1134 inevitably increases, so that the capillary forces are different and the flow velocities are different. The liquids with different flow velocities arrive at the gathering position 1139 at different times, resulting in mutual impact. In addition, the liquid is divided by the choke elements and flows around the choke elements in a changing manner, and the change in direction can also have the effect of mixing liquids.

The above is a mixing unit based on liquid division and gathering. In some embodiments, the channel where the choke elements are arranged is relatively wide on the whole, but the liquid is divided by the choke elements, and the channel at gathering areas becomes narrow, which is achieved by arranging some protrusions 11313 in the channel. At the gathering areas, the size of the channel decreases, and the flow velocity increases. Thus, when the liquid flows to the downstream choke element for division, as the liquid comes into contact with the choke element, a larger impact force can be formed, thereby achieving a better mixing effect. Therefore, with these protrusions, the size of the channel is changed, for example, the width is reduced, so that the capillary force is increased at the time of gathering, and the flow velocity is increased. When the liquid with an increased flow velocity flows to the next division element or the next mixing unit, for example, 1136-1, the liquid can impact the division element, so that the liquid is divided, thereby enhancing the mixing effect. The overall shape of the channel is formed by the shapes of walls 11314, 1137 forming the channel. The height of the walls defines the height of the channel, the distance between the walls defines the width of the channel, and the division element defines the widths of the divided channels., thereby changing the capillary force. As can be seen from FIG. 10A, both the width and the height of the channel are variable, and the capillary force is also variable. The channel is formed by the wall 11314 and the wall 1137, and the height of the walls and the width of the channel define the size of the channel together. One or more units which divide and gather the liquid through the choke element 1136 may be arranged in the mixing area. The mixture of the liquid sample is divided and gathered many times, so that the liquid can be thoroughly mixed in the mixing area with a shorter flow route. Similarly, another choke element 11312 is arranged upstream of the choke element 1136 in the channel such that the liquid is divided into two flow paths by the two sides of the circular choke element, and then the two flow paths become one flow path at the downstream gathering area 1133, thereby forming another mixing unit. Therefore, the essence of the mixing unit is the process in which the liquid is divided and then mixed in the mixing unit. In a specific implementation, the number of the mixing units may be determined by the number of the division elements, and the mixing units are distributed in the mixing channel in sequence. There may be one or more mixing units, for example, 1, 2, 3, 4, 5, 6, 7, 8, 9, 10, 11, 12, 15, 20, 25, 30 or 31 mixing units. In some embodiments, the number of folds of the mixing channel is reduced, the length of the mixing channel is reduced, and the size of the chip is reduced, so that the liquid sample and the reagents are mixed in another way.

In some embodiments, similar dried reagents, which may be the same as or different from the reagents in the dried reagent area, may be arranged in the mixing channel. For example, the dried reagents are arranged in the front area of the inlet 2117 of the mixing area, for example, on a side wall 1132-2 of the division element 1132-1, or on a surface 1132-3 of the divided channel or in the downstream gathering area 1133, so that the liquid sample firstly dissolves the dried reagents at these places in the mixing area. Since the flow of liquid forms turbulence, vortices and impact, the dried reagents can be quickly taken away by the liquid sample and then mixed with the liquid sample through the downstream mixing units. In this implementation, the dried reagent area may be omitted, so that the liquid sample directly enters the mixing area and the dried reagents are dissolved and mixed in the mixing area. Of course, in other embodiments, as shown in FIG. 8, the mixing area only allows the sample to be thoroughly mixed, contact and react with the dried reagents dissolved in the sample, and the dried reagents are not arranged in the mixing area in advance.

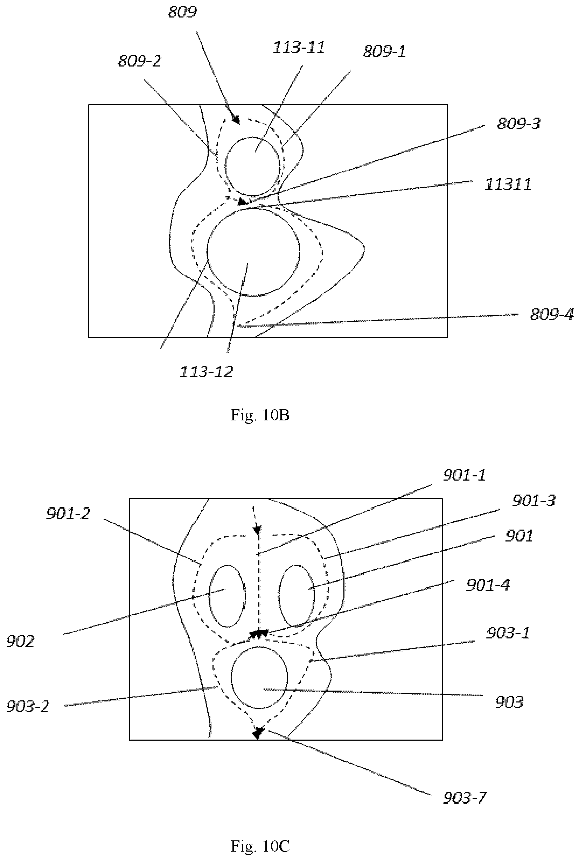

The liquid may be divided by the division elements into two paths or multiple paths, or divided and gathered repetitively, or some of the paths are gathered and then divided. Alternatively, in the gathering area, the liquid may be formed by the divided liquids in multiple paths gathering for multiple times or at different times. As shown in FIG. 10B, a first choke element 113-11 is arranged in the channel in the mixing area, and a second choke element 113-12 is arranged at an interval from the first choke element. The two choke elements have different sizes and an interval therebetween. In this way, the liquid sample 809 flowing from upstream to downstream is divided by the first choke element 113-11 into two flow paths 809-1, 809-2. Then, a flow path of liquid at the interval 11311 between the first and second choke elements is gathered with the flow path 809-1 at an intersection 809-3 and then continues flowing forward so as to be gathered with the flow path 809-2 at a gathering area 809-4. As shown in FIG. 10C, two choke elements 901, 902 are arranged side by side in the mixing area. The two choke elements form three flow paths 901-2, 901-4, 901-3. The three flow paths of liquid can be gathered at the gathering area 901-4, and immediately divided into two paths 903-1, 903-2 by a third choke element 903, and then, the two flow paths continue flowing and are gathered at a downstream gathering area 901-7. That is, the liquid can flow in a plurality of divided paths at one time, and any two flow paths can be gathered and then divided again. The purpose of this arrangement is to prolong the flow path of the liquid and make the liquid divided and gathered within a limited length of the channel so as to achieve thorough mixing. These division elements are typically solid, and the liquid will naturally become separated when it meets the solid. In some embodiments, as shown in FIG. 10A, the choke element 11312 has a height and a surface 1132-1, and the channel wall adjacent thereto also has a height and a surface. The inner surface of the top cover 105 may be bonded and sealed with a surface 11315 of a wall of the bottom cover to form a channel. In the sealed channel, the surface 1132-1 of the choke element is also bonded and sealed with the inner surface of the top cover 105, so that there are only two divided flow paths. Of course, if the height of the choke element 11312 is less than the height of the channel, then a third flow path is defined in the channel, which is the gap between the surface of the choke element 1132-1 and the inner surface of the top cover 105. The so-called "choke element" here has the function of dividing the liquid, and also has the functions of preventing the liquid from flowing and reducing the flow velocity of the liquid. For example, when the sample is a whole blood sample, the liquid, which is relatively viscous itself, flows through the dried reagent area such that the dried reagents are dissolved therein, and then the liquid enters the mixing area. The viscous liquid does not flow easily, so the division function is provided to make the liquid flow slower. Of course, this division and gathering process functions to make the blood thoroughly contact and react with the dried reagents. The shape of the choke element is not particularly limited, which may be a circle, rhombus, ellipse, semicircle, triangle, rectangle or square, and the height or the thickness is not particularly limited either. The number of the mixing units in the channel is also not particularly limited, which may be 2, 3, 4, 10, 15 or 20. The shape, height and thickness of the choke element, and the number of the mixing units in the channel may be selected through specific testing based on the properties of the sample and the lower limit and number of the analytes.

The driving force for driving the liquid to flow in the mixing area can make the liquid flow downstream only by capillary action. Since the plurality of division units are arranged in the mixing area, the capillary action changes constantly along with the division of the choke elements, which can be set arbitrarily. Of course, the flow from the mixing area into the testing area and the waste liquid area can be completed by capillary action alone.

In summary, the size of the channel in each mixing unit of the mixing area is changeable, and the size of each liquid flow path flowing through the channel is changeable, which leads to the change in the capillary action, so that the flow velocity of the liquid in each flow path may be different. The channel where the mixing area is located is divided by the choke elements into a plurality of small channels, and the change in size of these small channels leads to the change of capillary force and the change of the flow path, so that the driving force for mixing the liquid sample is formed in this state.

However, only by the capillary force, the capillary force of the mixing area itself and the capillary force of the testing area are different due to the structural arrangement of the channel (width, whether choke elements are contained), and different capillary forces lead to different flow velocities of the liquid. How to make the liquid flow through the testing area 114 at a stable speed needs to be considered. The second receptors are immobilized in the testing area, and the immobilized second receptors are used for capturing the complex formed by the first receptor and the analyte. If a plurality of testing units are arranged, each testing unit corresponds to one analyte It is expected that the flow velocity of the liquid in the testing area is stable in each testing unit, which can make the testing more accurate and avoid the test abnormalities and errors caused by the flow velocity. In some testing units, the flow velocity is higher, and in some testing units, the flow velocity is lower. Different times at which the immobilized second receptors capture the complexes lead to testing deviation between the testing units. Theoretically, it is expected to make each testing unit contact the liquid sample at the same time.

However, due to different requirements for sample treatment stages (the mixing area is to enhance binding of the first receptor to the analyte, and the testing area is to make the immobilized second receptor capture the complex) and mechanical errors in design, it is difficult to make the flow velocity in the mixing area consistent with that in the testing area. Moreover, the liquid in the mixing area flows disorderly along multiple flow paths in constantly changing directions at unstable flow velocities. However, in the testing area, it is expected that the liquid flows orderly at a stable flow velocity, the flow direction is substantially unchanged, the flow path is along a single direction, and the flow is substantially standard laminar flow.

In order to alleviate this problem, in some embodiments, a transition area 119 is arranged between the outlet 113-1 of the mixing area 113 and the inlet 113-4 of the testing area. The transition area changes the flow property (non-laminar flow) of the liquid required by the mixing area into the flow property (laminar flow) required by the testing area, and also changes the flow velocity required by the mixing area into the flow velocity required by the testing area. In order to enhance the mixing effect, it is expected that turbulence or vortices are formed in the mixing area to enhance the mixing effect, and that the liquid flows mainly in a laminar flow manner in the testing area to reduce turbulence or vortices. Therefore, the purpose of arranging the transition area 119 is to gradually change the flow velocity of the liquid flowing out of the mixing area to the flow velocity required by the testing area, and change the non-laminar flow manner (not mainly based on laminar flow) in the mixing area into the manner mainly based on laminar flow, and the purpose of the mixing area is to change the disorderly flow of liquid in the mixing area into an orderly and stable flow required by the testing area.

For example, if the flow velocity in the mixing area is relatively higher and the flow velocity in the testing area is relatively lower, then it is expected to gradually reduce the capillary force through the transition area 119 so as to gradually reduce the flow velocity, and then the flow velocity in the testing area is kept stable. Conversely, if the flow velocity in the mixing area is relatively low and the flow velocity in the testing area is relatively high, then it is expected to gradually increase the capillary force through the transition area so as to gradually increase the flow velocity, and then the flow velocity in the testing area is kept stable.

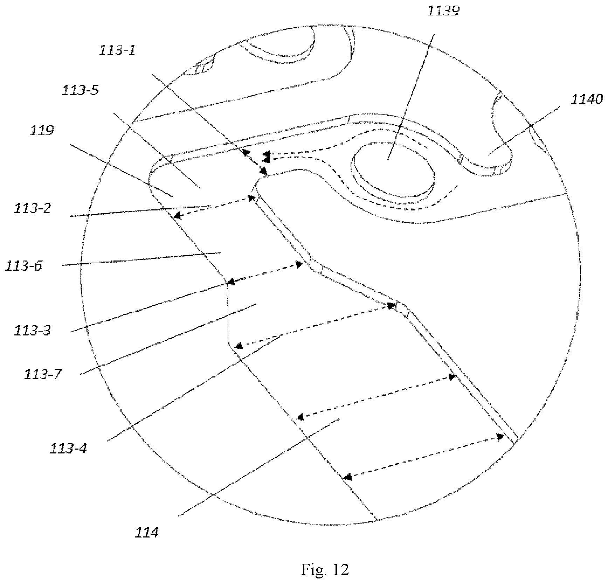

As can be seen from FIG. 12, in some embodiments, in the testing area, the width of the channel is relatively large, the capillary force is relatively small, and it is expected that the flow velocity of the liquid is reduced in the testing area. In the mixing area, since the channel is divided by the choke elements into a plurality of small microchannels, the capillary force is relatively large on the whole, and the flow velocity is relatively large, so it is expected to gradually reduce the flow velocity through the transition area such that the flow velocity in the testing area is stably kept on a low flow velocity level (relative to the mixing area). Therefore, in some embodiments, the transition area 119 is arranged at the outlet of the mixing area, and the flow direction of the liquid also substantially changes. The transition area connects the mixing area with the testing area. The width of the channel of the transition area gradually increases to be the same as the width of the channel in the testing area. The increase in width leads to the gradual decrease in capillary force and thus the decrease in the flow velocity of liquid, so that the flow velocity of the liquid in the testing area is more stable and kept at a relatively low level. In addition, the channel with the increased width gradually changes the liquid from non-laminar flow into laminar flow, so that the liquid flows in the testing area at an appropriate flow velocity in a laminar flow manner.

As shown in FIG. 12, the outlet 113-1 of the mixing channel is the last place where the divided flow paths are gathered and also the place where turbulence or vortices occur. If the outlet is directly connected to the testing area 114, the liquid directly flowing to the testing area is not substantially laminar, and there are still turbulence, eddies or vortices inside the liquid, which makes the flow of liquid in the testing area not heterogeneous. In some embodiments, a first transition area 113-5 is arranged at the outlet 113-1. The first transition area 113-5 directly changes the flow direction of the liquid from leftward (abscissa) to downward (ordinate). The corner actually functions to reduce the flow velocity of the liquid, change the flow direction of the liquid and also improve the turbulence and vortices inside the liquid. In some embodiments, a second buffer area 113-6 is arranged. The buffer area has a smaller width than the outlet 113-1 of the mixing area and gradually decreased capillary force, so that the flow velocity of the liquid is further reduced. The overall width of the buffer area is smaller than the width of the testing area. In other embodiments, a third transition area 113-7 is arranged. An outlet 113-4 of this area is also the inlet of the testing area, and has basically the same width as the testing area. In this way, the flow velocity of the liquid is gradually reduced, so that the liquid flows into the testing area at a more stable flow velocity. In some embodiments, the third transition area has an increasing width and decreasing capillary force. The width may increase gradually or sequentially, so that the capillary action decreases gradually, and the flow velocity of the liquid gradually decreases. Then, the liquid flows through the testing area at a stable flow velocity. These buffer areas arranged can not only reduce the flow velocity, but also make the disorderly liquid flow orderly.

The "disorderly" here means that the flow path, flow direction or flow velocity of the liquid is changing, and the "orderly" means that the flow path and the flow direction are unchanged and the flow velocity is stable. The flow of liquid in the mixing area is disorderly, and it is expected that the flow of liquid in the testing area is orderly.

The channel of the testing area has a constant width and constant capillary force, so that the liquid flows in the testing area at a stable flow velocity, thereby improving the accuracy of testing. It can be understood that in some tests, if the flow velocity in the mixing area is too low and it is expected that the flow velocity in the testing area is higher, then the width of the microchannels can be gradually reduced, so that the capillary force is gradually increased. Thus, the flow velocity is gradually increased, so that the liquid can flow in the testing area at a higher velocity.

By using the mixing units of the invention, the liquid sample and the reagents in the dried reagent area can be homogeneously distributed within a limited channel length. Through testing, among different quantities of unit volume samples, the quantity of the first receptor with the marker distributed in each unit volume is basically the same. For example, each unit milliliter or unit microliter carries the first receptor (i.e., antibody) with the marker in the same order of magnitude, for example, 10-1000 or 500-10000. However, in the case where the serpentine folded channel as shown in FIG. 19 is used to mix the same sample passing through the dried reagent area, the quantities of the first receptor with the marker in different unit volumes vary greatly. The quantity in some unit volumes is 1 to 5 times that in another unit volume, showing nonuniform distribution. In this case, since the flow of liquid is laminar in the downstream testing area, the test result in the testing area may be invalid in some cases, or part of the testing unit contains the marker and part of the testing unit is blank. Thus, only by constantly increasing the length and the number of folds of the serpentine channel can the purpose of uniform mixing be achieved.

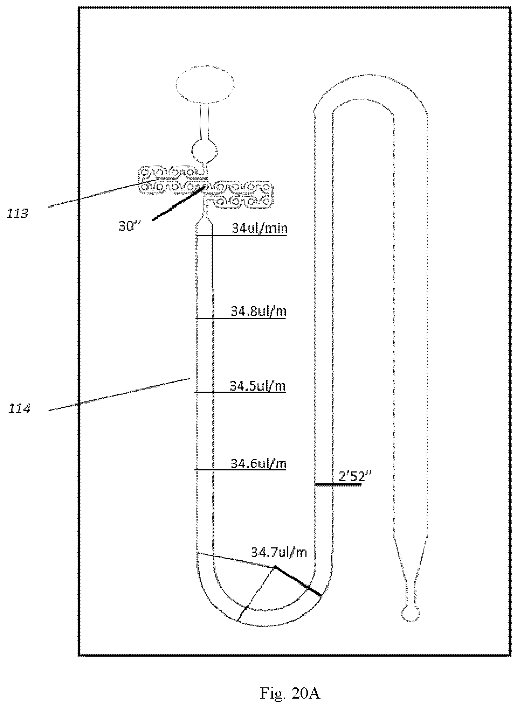

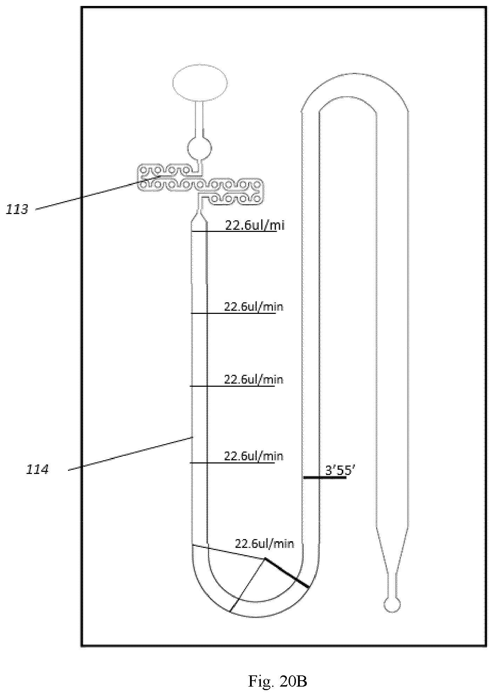

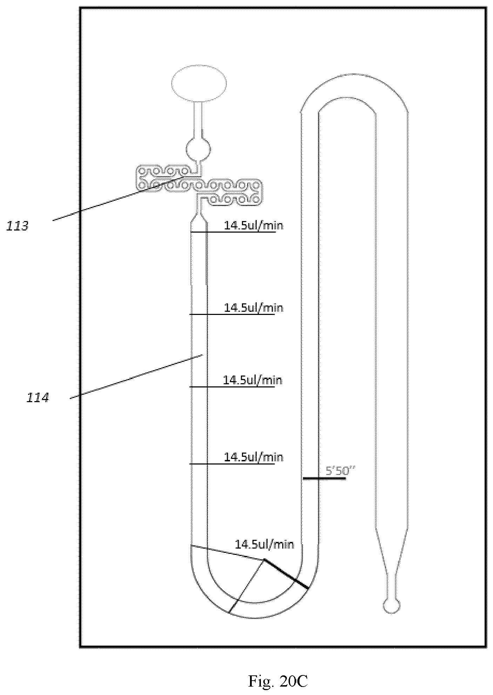

For example, as shown in FIG. 20C, after the liquid flows out of the mixing area 113, it enters the testing area for testing the flow velocity. When the liquid flows only by capillary action, in a case where the whole testing area has a length of 2 centimeters, a height of 90 microns and a width of 40 microns, the flow velocity is maintained at 14.5 microliters per minute, which is obtained by testing the distance of flow of liquid in unit time (minute) or the flow rate (the liquid generally flows along the channel while filling the whole testing area) of liquid in unit time. It takes about 5.50 minutes for the liquid to flow to the tail end. As shown in FIG. 20B, under the action of the pump (external driving force), the flow velocity in the testing area can be increased, but the flow velocity is still stable. The pump is started when the liquid enters the mixing area 113, such that the flow rate is stably maintained at 22.6 microliters per minute. This may be because the transition area 119 arranged between the mixing area and the testing area allows the flow of liquid to gradually change from non-laminar flow to laminar flow, thus realizing stable transition of flow velocity. For another example, as shown in the test result of FIG. 20A, it takes 30 seconds for the liquid to flow through the mixing area, it takes 2 minutes and 52 seconds for the liquid to flow to the designated position in the testing area, and the flow velocity in the testing area 114 is maintained within a stable range. The test is conducted according to a specific design manner of the invention, and the accompanying drawing is only the description of one schematic view.

Underline: First receptor and second receptor

In some embodiments, the dried reagent area treats one or more first receptors which can directly or indirectly bind to the analyte in the sample. The first receptors here may be antibodies, antibody fragments, antigens or antigen fragments, nucleic acids or nucleic acid fragments, or polypeptide fragments. The first receptors can also specifically bind to the analyte directly or indirectly. The first receptors here may also be antibodies, antibody fragments, antigens or antigen fragments, nucleic acids or nucleic acid fragments, or polypeptide fragments. In some embodiments, the second receptor includes markers which can be directly or indirectly connected or coupled with the first receptor. A second receptor is immobilized in the testing area. The second receptor, which cannot flow with the liquid, is used to capture the complex formed by the first receptor and the analyte, and the quantity of the analyte is expressed according to the quantity of the complex. For example, when the antibody (the analyte) in the liquid sample is tested, the first receptor may be the antigen, and the second receptor may be the antibody of the antigen in the liquid sample. In some embodiments, the first receptor may be the first antibody of the antigen in the liquid sample, and the second receptor may be the second antibody of the antigen in the liquid sample. When the analyte in the test sample is the antigen, the first receptor can be the first antibody of the antigen, and the second receptor can be the second antibody of the antigen. These first antibodies can be connected with the marker, and the marker can be used as a signal substance to show the presence or absence or quantity of the analytes in the liquid sample. In some embodiments, the first receptor or the first receptor with the marker can flow with the liquid, and the second receptor in the testing area is immobilized and cannot flow with the liquid.

Underline: Antibody

The antibody used in the invention can be any antibody capable of specifically binding to the analyte in the liquid sample. The antibody that can be used as a binding agent can be any antibody known to those skilled in the art. The "antibody" can be an immunoglobulin molecule and an antigen binding part of the immunoglobulin molecule, that is, a molecule including an antigen-binding site that specifically binds to an analyte, an analyte analog or a ligand ("immune reaction"). This term also includes derivatives of antibodies in which the binding ability is retained, and also includes any protein containing a binding domain that is homologous or largely homologous to the binding domain of an immunoglobulin. These proteins may originate from natural substances, or they may be partially or fully synthesized. An antibody may be monoclonal or polyclonal. An antibody may be a member of any immunoglobulin type, including any human immunoglobulin type: IgG, IgM, IgA, IgD, IgG, and IgE. An "antibody fragment" is a derivative of an antibody or a portion of an antibody that is less than the full length. The antibody fragment can retain at least one significant site of the binding ability of the full-length antibody. Examples of the antibody fragment include an antigen-binding fragment (Fab), an Fab', an F(ab')$_2$, a single-chain variable fragment (scFv), a variable fragment (Fv), a disulfide-stabilized Fv (dsFv) dimer, and an Fd fragment, but are not limited to the above. The antibody fragment can be generated by any means. For example, the antibody fragment can be generated by enzymatic or chemical cleavage of a complete antibody, or can also be generated by recombination of genes encoding partial antibody sequences. In other words, the antibody fragment can be generated by partial or complete recombination. The antibody fragment can be any single-chain antibody fragment. In other words, the antibody fragment can include a plurality of peptide chains linked to each other, for example, by disulfide bonds. The antibody fragment can also be any one of multi-molecular complexes. One functional antibody fragment typically includes at least about 50 amino acids, while more antibody fragments typically include at least about 200 amino acids. Single-chain Fvs (scFvs) are recombinant antibody fragments consisting only of a variable region of light chain ($V_L$) and a variable region of heavy chain ($V_H$) covalently bound to each other in a polypeptide chain. One of $V_L$ and $V_H$ has an amino terminal region. The length and composition of polypeptide chains are variable, and their length can bridge two variable domains to each other without significantly affecting the arrangement of atoms. Polypeptide chains are typically composed mainly of glycine and serine residue extensions, with some glutamic acid and lysine residues scattered to increase their solubility. The "dimer" refers to a bipolymer of single-stranded Fvs. The monomers of the dimer typically include peptide chains that are shorter than those of most single-chain Fvs, and they show a tendency to form the bipolymer.

The "Fv" fragment consists of one $V_H$ and one $V_L$ domain non-covalently linked to each other. The term "dsFv" here refers to an Fv including an intermolecular disulfide bond that stabilizes a $V_H$-$V_L$ pair. The "F(ab')" fragment is a fragment of an antibody that is essentially identical to the fragment obtained by digesting an immunoglobulin (usually IgG) with pepsin at pH 4.0--4.5. This fragment can also be synthesized by recombination. The "Fab‴" fragment is an antibody fragment that is essentially identical to the fragment obtained by reducing the disulfide bond linking the two heavy chains on the F(ab') fragment. The Fab' fragment can also be synthesized by recombination. The "Fab" fragment is an antibody fragment that is essentially identical to the fragment obtained by digesting an immunoglobulin (usually IgG) with papain. The Fab fragment can also be synthesized by recombination. The heavy chain fragment on the Fab fragment is the Fd fragment.

The antibody here can also be any kind of antibodies in the analyte, and these antibodies can also be used as the analyte.

Nucleic acids

In some embodiments, the device of the invention can be used to test the quantity, presence or absence of nucleic acids in the liquid sample in a high-throughput way. Generally, the first receptors include objective nucleic acids with substantially complementary sequences; the sequences are immobilized onto the testing area, but the objective nucleic acids or the target nucleic acids (which can also be called analytes) exist in the liquid sample; alternatively, the amplified products of the objective nucleic acids or the target nucleic acids in the liquid sample cause the complementary sequences to bind to the objective nucleic acids or the amplified nucleic acids form a conjugate; then, a probe is allowed to bind to the objective nucleic acids or the target nucleic acids and can include the marker, for example, fluorescent makers; the quantity of the objective nucleic acids can be identified through the quantity of the fluorescent markers on the probe. Amplification here may be any form of amplification, for example, PCR amplification, isothermal amplification, RPA technology or RAA technology; primers and necessary reagents to complete amplification are needed for nucleic acid amplification. All these methods are conventional technologies.

The term "substantially complementary", when used to define an amino acid sequence or a nucleic acid sequence, means that a specific target sequence such as an oligonucleotide sequence is substantially complementary to all or a part of selected sequences, and therefore will specifically bind to a part of mRNA to encode the selected sequences. Therefore, generally, the sequences will be highly complementary to the "target" sequence of mRNA, and the complementary part of the sequences will have no more than about 1, about 2, about 3, about 4, about 5, about 6, about 7, about 8, about 9, or about 10 base mismatches. In many cases, it may be desired that the sequences are exactly matched, that is, the sequences are completely complementary to those specifically binding to the oligonucleotide, so zero mismatch exists in a complementary segment. Therefore, generally, highly complementary sequences will quite specifically bind to the target sequence region of mRNA, thus effectively reducing and/or even inhibiting translation of the target sequence of mRNA into a polypeptide product.

A substantially complementary nucleic acid sequence will be more than about 80% complementary (or "% exact match") to a corresponding nucleic acid target sequence that specifically binds to the nucleic acids, and more preferably, will be more than about 85% complementary to a corresponding target sequence that specifically binds to the nucleic acids. In some aspects, as mentioned above, it will be desirable to have even more substantially complementary nucleic acid sequences in practices of the invention; in such cases, the nucleic acid sequences will be more than about 90% complementary to the corresponding target sequences that specifically bind to the nucleic acids, and in some embodiments, the nucleic acid sequences can be more than about 95% complementary to the corresponding target sequences that specifically bind to the nucleic acids, and the nucleic acid sequences can be even up to and including about 96%, about 97%, about 98%, about 99% and even about 100% exactly complementary to all or a part of the target sequences that specifically bind to the designed nucleic acids.

The percentage of similarity or complementarity of any disclosed nucleic acid sequence can be determined, for example, by comparing sequence information using GAP computer program version 6.0 available from the University of Wisconsin Genetics Computer Group (UWGCG). Comparison methods of Needleman-Wunsch algorithm (1970) are adopted in the GAP program. In short, the GAP program defines similarity as the number of similar comparison symbols (namely, nucleotides or amino acids) divided by the total number of symbols in the shorter sequence of two sequences. The preferred default parameters of the GAP program include: (1) a unary comparison matrix of nucleotides (including a value of 1 indicating identity and a value of 0 indicating non-identity), and a weighted comparison matrix of Gribskov and Burgess (1986); (2) a penalty score for each gap being 3.0 and an extra penalty score for each symbol in each gap being 0.10; and (3) no penalty score for an end gap.

Naturally, the invention further includes nucleic acid segments that are complementary, essentially complementary and/or substantially complementary to at least one or more specific nucleotide sequences specifically set forth herein. A "complementary" nucleic acid sequence is a nucleic acid sequence that can be subjected to base pairing according to standard Watson-Crick complementary rules. As used herein, the term "complementary sequence" means a substantially complementary nucleic acid sequence, and can be evaluated through comparison of same nucleotides described above or defined as being hybridized with one or more specific nucleic acid segments disclosed herein under more stringent conditions, such as those just described above.

As used herein, the term "virtually excluding" or "substantially excluding" in relation to the amount of components preferably means that the composition includes a compound of less than about 10% by weight, preferably less than about 5% by weight, and more preferably less than about 1% by weight. In preferred embodiments, these terms mean less than about 0.5% by weight, less than about 0.1% by weight or less than about 0.01% by weight.

The probes and primers used in the invention may have any suitable length. The numerical values are assigned to the sequence, for example, 1 denotes a first residue and 2 denotes a second residue, so an algorithm can be proposed to limit all probes or primers included in a given sequence: n to n+y, where n is an integer from 1 to the last number of the sequence, y is the length of the probe or primer minus 1, and n+y does not exceed the last number of the sequence. Therefore, for a probe or primer with 25 base pairs (namely, a "25-mer"), sets of probes or primers correspond to bases 1 to 25, bases 2 to 26, bases 3 to 27, bases 4 to 28, and the like over the entire length of the sequence. Similarly, for a probe or primer of 35 base pairs (namely, a "35-mer"), an exemplary primer or probe sequence includes, but is not limited to, a sequence corresponding to bases 1 to 35, bases 2 to 36, bases 3 to 37, bases 4 to 38, and the like over the entire length of the sequence. Similarly, for a 40-mer, such probes or primers can correspond to nucleotide sequences from a first base pair to a 40th base pair, from a second base pair to a 41st base pair, from a third base pair to a 42nd base pair, and the like; for a 50-mer, such probes or primers can correspond to nucleotide sequences from a first base pair to a 50th base pair, from a second base pair to a 51st base pair, from a third base pair to a 52nd base pair, from a fourth base pair to a 53rd base pair, and the like.

The term "substantially corresponding to", "substantially homologous" or "substantially identical" as used herein indicates the characteristics of a nucleic acid sequence or an amino acid sequence, where the nucleic acid sequence or the amino acid sequence selected has at least about 70% or about 75% sequence identity when compared with the reference nucleic acid sequence or the reference amino acid sequence selected. More generally, the sequence and the reference sequence selected will have sequence identity of at least about 76%, 77%, 78%, 79%, 80%, 81%, 82%, 83%, 84% or even 85%, and more preferably at least about 86%, 87%, 88%, 89%, 90%, 91%, 92%, 93%, 94% or 95%. Still more preferably, highly homologous sequences often share more than at least about 96%, 97%, 98% or 99% sequence identity between the selected sequence and the reference sequence with which it is compared.

Testing area

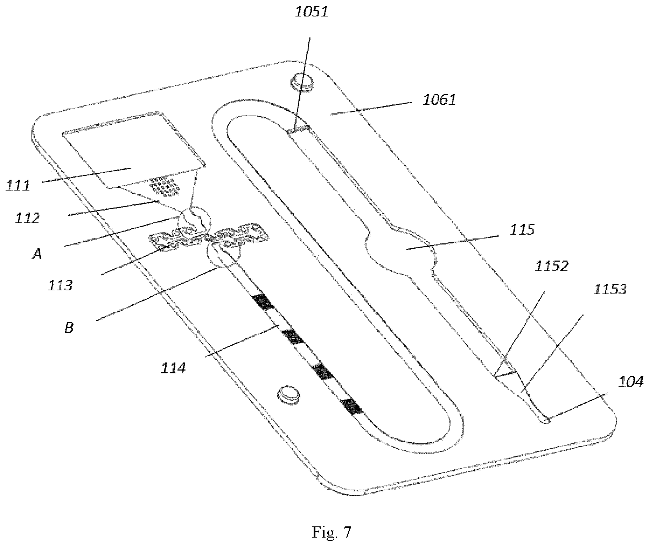

In some embodiments, a testing area is arranged downstream of the mixing area. The testing area is provided with at least one testing units, and each testing unit is used for testing one analyte. A second receptor is immobilized in the testing area and used for capturing the complex (if any) flowing out of the mixing area, and the marker on the complex is used to indicate the presence or quantity of the analyte in the liquid sample. As shown in FIG. 7, the testing area 114 is an area where the liquid flowing downstream from the mixing area 113 is tested, and of course, it may be a place where the liquid passing through the mixing area and the transition area flows. The testing area is arranged before the waste liquid area 115 and the mixing area 113. In some embodiments, the testing area includes one or more testing units, for example, 4 testing units 1141, 1142, 1143, 1144. The second receptor is immobilized on each testing unit, and the quantity or type of second receptor immobilized on each testing unit may be different. In some embodiments, the second receptor is immobilized on the testing unit and used for capturing the complex in the liquid sample, and the quantity of the marker on the complex is detected to indicate the quantity of the analyte. If the marker is colored, then the test result in the testing area is read through a window 103 in the upper cover. If the marker is colorless and invisible, the test result, such as fluorescence intensity, is read by a machine. The testing area may have a diameter of 80 microns and a height of 40 microns, and the interval between the testing units may be 60 microns. Within these ranges, the testing area also has capillary force.

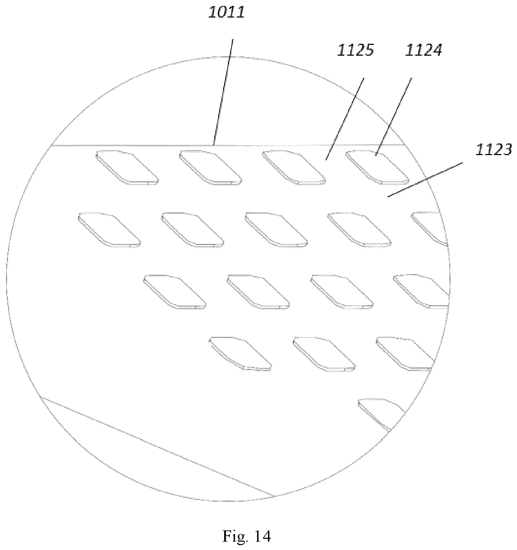

In some embodiments, the testing unit has a rough surface, which is configured to make the second receptor immobilized or unable to flow with the liquid. As shown in FIG. 14, in some embodiments, the rough surface is provided with a plurality of small protrusions 1147 which are configured to immobilize the second receptor. In some embodiments, the testing area is a channel with a constant size, so that the liquid can flow in this area by the capillary force of the channel. However, the capillary force is substantially constant, so that the liquid can flow in the channel in a substantially "laminar flow" manner, and the liquid can flow through the testing area at a fixed or stable flow velocity, thereby realizing testing of the analyte in the liquid sample. Here, the substantially constant capillary force can be realized by making the size, cross-sectional area and width (the height is constant) of the microchannels in the testing area constant.

In some embodiments, the testing area is configured to receive the sample from the mixing area. This liquid sample may contain reagents in the dried reagent area, or may be just the liquid sample. If the reagents arranged in the dried reagent area are completely and substantially taken away by the liquid sample and flow downstream and there is an excess of liquid sample (which substantially does not contain the reagents in the dried reagent area) flowing through the dried reagent area, the choke area (if any), the transition area (if any) between the choke area and the mixing area, the mixing area and the transition area (if any) between the mixing area and the testing area and entering the testing area, then a part of the sample flowing through the testing area contains the dried reagents, and the other part of the sample does not contain the reagents in the dried reagent area. Generally, the sample containing the dried reagents in the dried reagent area flows through the testing area first, and the sample that does not contain the reagents in the dried reagent area flows through the testing area later. The sample that does not contain the reagents in the dried reagent area actually functions to flush the testing area, for example, taking away some complex not captured by the second receptor and other byproducts, which reduces the background interference and improves the detection sensitivity and accuracy. Therefore, an excess of liquid sample may be added, and the quantity of the dried reagent, for example, the first receptor, in the dried reagent area is less than the quantity of the analyte in the liquid sample. In this way, there is a surplus of liquid sample that basically does not participate in the reaction but functions to flush the background.

Waste liquid area

In some embodiments, a waste liquid area 115 is further arranged downstream of the testing area and configured to collect or store the waste liquid from the testing area, including the liquid sample. In order to better collect and store the waste liquid, a volume of the waste liquid area is greater than a volume of the liquid sample. In order to facilitate the liquid to enter the waste liquid area, the waste liquid area may also have capillary force, but the capillary force of the waste liquid area should be less than the capillary force of the testing area, or the length of the testing area should be greater than the length of the waste liquid area. In this way, when the liquid sample enters the waste liquid area, at least the capillary force in the waste liquid area cannot accelerate the flow of liquid in the testing area. In some embodiments, in order to reduce the capillary force of the waste liquid area, the depth of the waste liquid is greater than the depth of the testing area, or the bottom of the channel in the waste liquid area is higher than the inner surface of the upper cover. In this way, the waste liquid area can collect the waste liquid better.

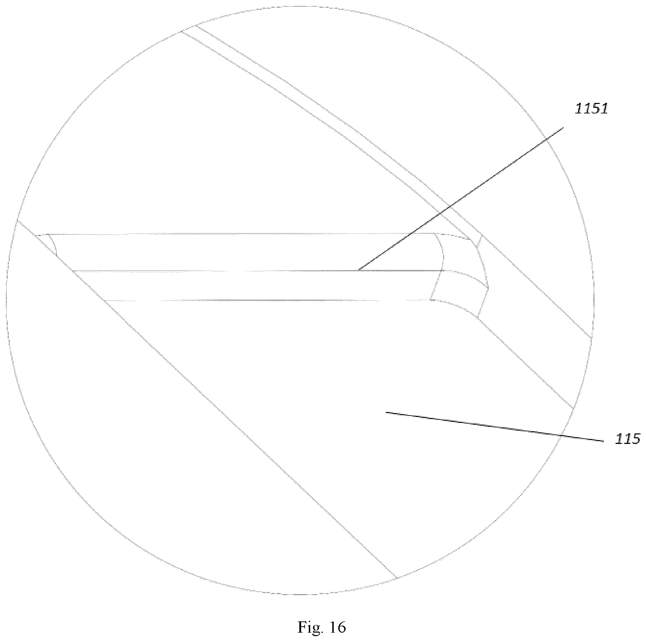

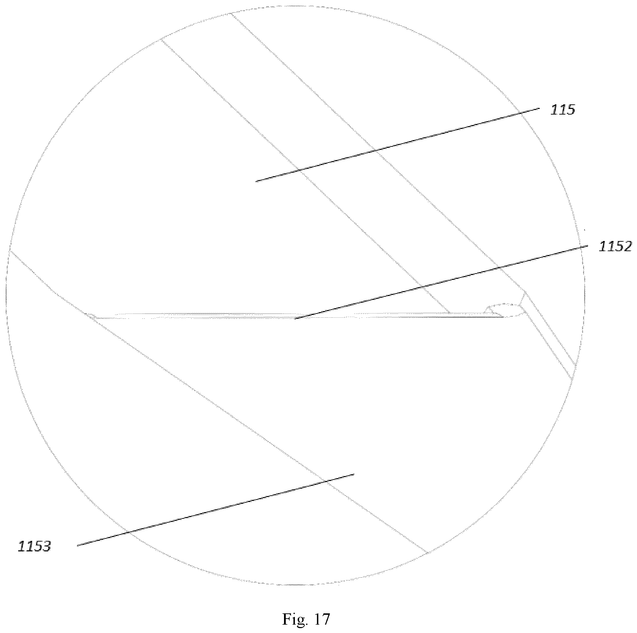

In some embodiments, a waste liquid area 115 is further arranged downstream of the testing area. The waste liquid area has two ends which are respectively connected to the testing area and the outlet 1151. For example, as shown in FIG. 16 to FIG. 18, at one end 1151 of the waste liquid area 115 connected to the testing area, a sink is formed at this end, which makes the entire waste liquid area sink relative to the testing area. In this way, after the liquid flows into the waste liquid area, the liquid is less prone to flowing back. Similarly, the other end 1152 of the waste liquid area 115 is connected to the outlet 104. This end 1152 is not directly connected to the outlet 104, but there is a section of channel 1153 in the middle. In this way, even if the waste liquid flows into the waste liquid area 115, it will not directly flow to the outlet 104, otherwise the liquid will enter the pump and contaminate the pump.

Action of pump

In some embodiments, an outlet 104 of the microchannels is arranged in the chip. The outlet is connected with a pump, and the flow of liquid in the microchannels is pushed by the action of the pump. It can be understood that if the microchannel chip is disposable, the analyte can be tested only by the capillary force of the microchannels and without the action of the pump. In this case, the outlet 104 functions to discharge gas when the liquid flows in the microchannels. If the outlet is connected with the pump, not only the gas can be discharged, driving force for pushing the liquid to flow in the microchannels can be provided. Actually, if the pump exists, the liquid can flow in the microchannels by capillary action alone, or by the action of the pump alone or by the action of both the pump and the capillary force. When the liquid flows in the channel by capillary force, the pump can work at any time to apply a subatmospheric or negative pressure or a positive pressure in the channel. The subatmospheric or negative pressure can accelerate the flow of the liquid, and the positive pressure can make the liquid flow back.

For example, once the liquid sample is added to the device, the pump can be started to apply a stable subatmospheric or negative pressure. In this case, the subatmospheric or negative pressure drives the liquid sample to flow through the dried reagent area, the choke area, the mixing area and the testing area. Of course, the pressure is variable. When the liquid sample flows through the dried reagent area, in order to make the liquid sample stay in the dried reagent area for a longer time, the pressure in the channel can be slightly increased so as to prevent the liquid from continuing flowing or slow the flow of liquid. After a sufficient period of time, the subatmospheric or negative pressure is applied to accelerate the flow of liquid and allow the liquid to flow through the mixing area.

In some embodiments, although the mixing and flow of the liquid can be achieved in the mixing area by capillary force, the liquid is basically not laminar but non-laminar in the mixing area, and forming driving force required for mixing is a power-consuming process. In this case, the subatmospheric or negative pressure is applied by the pump to accelerate the flow of liquid in the mixing area, the formation of intense turbulence and vortices and the gathering of liquid, which can make the liquid mixed more thoroughly and accelerate the flow velocity of the liquid.

In some embodiments, using the action of the pump is an easy method to maintain a stable subatmospheric or negative pressure, and under a stable subatmospheric or negative pressure, it is expected that the flow velocity of the liquid flowing through the testing area is maintained stable or substantially constant. Therefore, in the invention, the channel is hydrophilically treated. It is found that the channel in the hydrophilically treated testing area can improve the flow velocity of the liquid and keep the flow velocity stable. The mixing channel and the testing channel are hydrophilically treated. In a case where a flow velocity of 8 microliters per minute is achieved under the control of the pump, the stability of the flow velocity of the liquid in the testing area is improved. When the mixing channel and the testing channel are not hydrophilically treated, the flow velocity changes when the liquid flows through different sections, i.e., the flow velocity gradually decreases from the beginning to the end. When the mixing channel and the testing channel are hydrophilically treated, the flow velocity still gradually decreases, but the difference between the flow velocity at the beginning and the flow velocity at the end becomes smaller, and the flow velocity is kept relatively stable.

There are some other embodiments as below is also the detailed embodiments in this application.

1. A microfluidic device for testing an analyte in a liquid sample, comprising:

a channel comprising an inlet and an outlet; and a dried reagent area and a testing area that are located in the channel, wherein the testing area is located downstream of the dried reagent area; and the inlet of the channel is configured to receive a liquid sample.

2. The microfluidic device according to clause 1, wherein the dried reagent area is located downstream of the inlet of the channel, and the dried reagent area comprises a liquid inlet and a liquid outlet.

3. The microfluidic device according to clauses 1-2, wherein the channel between the liquid inlet and the outlet has capillary force.

4. The microfluidic device according to clauses 1-3, wherein the capillary force of the liquid inlet of the dried reagent area is less than the capillary force of the liquid outlet, or the capillary force gradually increases from the liquid inlet to the liquid outlet of the dried reagent area.

5. The microfluidic device according to clauses 1-4, wherein the dried reagent area comprises a first receptor containing a marker, and the first receptor is capable of being moved with the flow of the liquid sample.

6. The microfluidic device according to clauses 1-5, wherein the dried reagent area is a funnel-shaped area having two ends, the end close to the inlet of the channel is treated with a dried reagent, and the end away from the inlet is directly or indirectly connected to the testing area.

7. The microfluidic device according to clauses 2-6, wherein a width of the inlet of the dried reagent area is greater than a width of the outlet.

8. The microfluidic device according to clauses 2-7, wherein a flow rate of the liquid per unit time at the inlet of the dried reagent area is greater than a flow rate of the liquid per unit time at the outlet.

9. The microfluidic device according to clauses 3-8, wherein a cross-sectional area of the inlet of the dried reagent area is greater than a cross-sectional area of the outlet.

10. The microfluidic device according to clauses 3-9, wherein the inlet of the dried reagent area is the inlet of the channel.

11. The microfluidic device according to clauses 1-10, wherein a sample channel is arranged upstream of the inlet of the channel, and capillary force of the sample channel is less than the capillary force of the inlet of the channel.

12. The microfluidic device according to clauses 11, wherein an inlet for adding the liquid sample is provided upstream of the sample channel.

13. The microfluidic device according to clauses 1-12, wherein a choke area is arranged downstream of the dried reagent area, and the choke area is capable of reducing a flow velocity or flow rate of the liquid in the channel from the inlet of the channel to the outlet of the dried reagent area.

14. The microfluidic device according to clauses 1-13, wherein a choke area is arranged downstream of the dried reagent area, and capillary force of the choke area is less than capillary force of the liquid outlet of the dried reagent area.

15. The microfluidic device according to clauses 13-14, wherein the choke area comprises a choke channel, and a widest distance of the choke area is greater than a maximum distance of the liquid outlet of the dried reagent area.

16. The microfluidic device according to clauses 13-15, wherein the choke area comprises a choke channel, and a cross-sectional area of a part of the choke channel is greater than a cross-sectional area of the liquid outlet of the dried reagent area.

17. The microfluidic device according to clauses 13-16, wherein the choke area comprises a choke channel, wherein a flow rate of the liquid per unit time in a part of the choke channel is less than a flow rate of the liquid per unit time at the liquid outlet of the dried reagent area.

18. The microfluidic device according to clauses 13-17, wherein the choke area comprises a hydrophobic area in which the flow velocity or flow rate of the liquid is reduced, so that the flow velocity or flow rate of the liquid flowing out of the outlet of the dried reagent area is reduced.

19. The microfluidic device according to clauses 1-18, wherein the device further comprises a mixing area, and the mixing area is located between the dried reagent area and the testing area.

20. The microfluidic device according to clauses 19, wherein the liquid in the mixing area flows in a substantially non-laminar flow manner.

21. The microfluidic device according to clauses 19-20, wherein a distance or time of the liquid flowing in the mixing area is greater than a distance or time of the liquid flowing in the dried reagent area or the testing area.

22. The microfluidic device according to clauses 19-21, wherein the liquid flows in the mixing area along two or more flow paths.

23. The microfluidic device according to clauses 19-22, wherein the liquid flows at different flow velocities, for different times, for different times or in different directions in each flow path.

24. The microfluidic device according to clauses 19-23, wherein the liquid is gathered together after flowing through different flow paths.

25. The microfluidic device according to clauses 19-24, wherein the mixing area comprises a choke element, and the choke element is capable of dividing the liquid in the mixing area so as to form the two or more liquid flow paths.

26. The microfluidic device according to clauses 22, wherein there is a liquid gathering area at tail ends of the different liquid flow paths.

27. The microfluidic device according to clause 20, wherein the non-laminar flow comprises turbulence or/and vortices.

28. The microfluidic device according to clause 19, wherein the mixing area contains at least one mixing unit, each mixing unit comprises one choke element, the liquid is divided into different liquid flow paths by the choke element, and the liquid flowing through different liquid flow paths is gathered together in the mixing unit to enter the next mixing unit.

29. The microfluidic device according to clause 28, wherein the plurality of mixing units are sequentially arranged from upstream to downstream from the outlet of the mixing area to the outlet of the mixing area.

30. The microfluidic device according to clause 28, wherein the choke elements may be in a shape of one or more of a circle, a square, an ellipse, a rhombus and a five-pointed star.

31. The microfluidic device according to clause 19, wherein a flow velocity or flow rate of the liquid in the mixing area is less than a flow velocity or flow rate of the liquid in the testing area.

32. The microfluidic device according to clause 19, wherein a flow velocity or flow rate of the liquid in the mixing area is greater than a flow velocity or flow rate of the liquid in the dried reagent area.

33. The microfluidic device according to clause 19, wherein a buffer area is arranged downstream of the mixing area, the testing area is arranged downstream of the buffer area, and the buffer area is capable of gradually reducing a flow velocity or flow rate of the liquid flowing out of the mixing area.

34. The microfluidic device according to clause 19, wherein a buffer area is arranged downstream of the mixing area, the testing area is arranged downstream of the buffer area, and a size of the channel in the buffer area gradually changes to be substantially the same as a size of the channel in the testing area.

35. The microfluidic device according to clause 32, wherein the buffer area comprises a plurality of buffer areas, and a first buffer area of the plurality of buffer areas is capable of changing a flow direction of the liquid.

36. The microfluidic device according to clause 35, wherein a second buffer area is further arranged downstream of the first buffer area, and a flow direction of the liquid in the second buffer area is the same as a flow direction of the liquid in the testing area.

37. The microfluidic device according to clause 19, wherein capillary force in the testing area is less than capillary force in the mixing area.

38. The microfluidic device according to clause 19, wherein a width of the testing area is greater than a width of the mixing area.

39. The microfluidic device according to clause 19, wherein a choke area is further arranged between the dried reagent area and the mixing area, a buffer area is arranged downstream of the choke area, and the mixing area is connected downstream of the buffer area.

40. The microfluidic device according to clause 39, wherein the liquid in the buffer area enters the mixing area after a flow direction of the liquid is changed.

41. The microfluidic device according to clause 19, wherein the mixing area comprises a liquid inlet and a liquid outlet, a flow direction of the liquid at the liquid inlet is perpendicular to a flow direction of the liquid in the dried reagent area, and a flow direction of the liquid at the liquid outlet is substantially perpendicular to a flow direction of the liquid in the testing area.

42. The microfluidic device according to clause 19, wherein the mixing area connects the dried reagent area with the testing area serpentine in a serpentine manner.

43. The microfluidic device according to clause 1, wherein the channel further comprises a waste liquid area, and the waste liquid area is located downstream of the testing area and close to the outlet of the channel.

44. The microfluidic device according to clause 43, wherein a depth of the channel in the waste liquid area is greater than a depth of the testing area.

45. The microfluidic device according to clause 1, wherein the testing area comprises one or more testing units, and a second receptor is immobilized in each of the testing units.

46. The microfluidic device according to clause 11, wherein a hole in communication with outside atmosphere is provided in the sample channel.

47. The microfluidic device according to clause 11, wherein a hole in communication with outside atmosphere is provided between a sample application opening and the inlet of the channel.

48. The microfluidic device according to clause 19, wherein the liquid flows in the dried reagent area, the mixing area and the testing area by capillary force.

49. The microfluidic device according to clause 19, wherein the liquid flows from the mixing area to the testing area by capillary force in combination with an action of the pump connected with the outlet.

50. The microfluidic device according to clause 1, wherein an inner surface of the channel in the testing area is hydrophilically treated.

51. The microfluidic device according to clause 13, wherein an inner surface of the channel in the choke area is treated with a hydrophobic reagent.

52. The microfluidic device according to clause 1, wherein the liquid sample comprises one of blood, urine and saliva.

53. A microfluidic device for testing an analyte in a liquid sample, comprising:
a channel containing a channel inlet and a channel outlet and having capillary force; and a dried reagent area, a mixing area and a testing area sequentially arranged from upstream to downstream between the inlet and the outlet, wherein the liquid flows in the dried reagent area and the testing area in a substantially laminar flow manner, and the liquid flows in the mixing area in a substantially non-laminar flow manner.

54. The microfluidic device according to clause 53, wherein a flow direction of the liquid in the mixing area changes, and a flow path of the liquid changes or a flow velocity or flow rate of the liquid changes.

55. The microfluidic device according to clause 54, wherein the mixing area comprises one or more mixing units, each mixing unit comprises one or more division elements, and the liquid is divided by the division elements in the mixing unit one or more times and then gathered together.

56. The microfluidic device according to clause 54, wherein the mixing area comprises one or more mixing units, each mixing unit comprises one or more division elements, and the liquid is divided by the one or more division elements in each mixing unit one or more times and then gathered together, and then flows into the next mixing unit.

57. A microfluidic device for testing an analyte in a liquid sample, comprising:

a microfluidic channel comprising a channel inlet and a channel outlet; and

a dried reagent area, a mixing area and a testing area between the inlet and the outlet, wherein the dried reagent area is located upstream of the mixing area, the mixing area is located downstream of the testing area, a sample channel is arranged upstream of the inlet of the channel, and a sample application opening is arranged upstream of the sample channel.

58. The microfluidic device according to clause 57, wherein the sample channel between the sample application opening and the inlet of the channel is provided with a vent hole in communication with the sample channel.

59. The microfluidic device according to clause 57, wherein capillary force of the sample channel is less than capillary force of the dried reagent area.

60. The microfluidic device according to clause 57, wherein a height of the sample channel is greater than or much greater than a height of the channel in the dried reagent area.

61. A method for testing an analyte in a sample, comprising:

providing a microfluidic device, comprising a dried reagent area, a mixing area and a testing area; and

adding a liquid sample to the microfluidic device, and allowing the liquid sample to enter the dried reagent area and then sequentially flow through the mixing area and testing area, wherein a flow velocity or flow rate of the liquid in the dried reagent area is allowed to be less than a flow velcotiy or flow rate of the liquid in the mixing area.

62. The method according to clause 61, wherein when the liquid enters the dried reagent area, the liquid is allowed to flow only by capillary force of a channel.

63. The method according to clause 61, wherein the flow velocity or flow rate of the liquid flowing through the mixing area is allowed to be greater than a flow velocity or flow rate of the liquid flowing through the testing area.

64. The method according to clause 61, wherein the liquid sample comprises whole blood, plasma or serum.

65. The method according to clause 61, wherein in a process of the liquid flowing through the mixing area, the flow velocity or flow rate of the liquid, a flow path of the liquid, or a flow direction of the liquid is allowed to change constantly.

66. The method according to clause 65, wherein the liquid is allowed to be divided at least one time in the mixing area, flow along the divided flow paths and then be gathered at least one time.

67. The method according to clause 63, wherein a transition area is arranged between the dried reagent area and the mixing area, and through the transition area, flow properties of the liquid are changed.

68. The method according to clause 67, wherein the flow properties comprise a flow direction, a flow velocity or flow rate and a flow path, and being laminar or non-laminar.

69. The method according to clause 68, wherein through the transition area, the flow velocity or flow rate of the liquid is gradually changed to be substantially the same as a required flow velocity or flow rate of the liquid in the testing area.

70. The method according to clause 68, wherein through the transition area, the flow velocity or flow rate of the liquid is gradually reduced to a required flow velocity or flow rate of the liquid in the testing area.

All patents and publications mentioned in the specification of the invention indicate that these are disclosed techniques in the art and can be used by the invention. All patents and publications cited herein are likewise listed in the references as if each publication is specifically and separately referenced. The invention described herein may be implemented in the absence of any one or more elements, and one or more limitations, which are not specifically stated herein. For example, the terms "comprising", "essentially consisting of" and "consisting of" in each example here may be replaced by the rest 2 terms. The term "a/an" here merely means "one", but does not exclude including 2 or more instead of including only one. The terms and expressions which have been employed here are descriptive rather than restrictive, and there is no intention to suggest that these terms and expressions in this description exclude any equivalents, but it is to be understood that any appropriate changes or modifications can be made within the scope of the invention and appended claims. It can be understood that the embodiments described in the invention are some preferred embodiments and features. Any person of ordinary skill in the art can make some modifications and changes according to the spirit of the description of the invention. These modifications and changes are also considered to fall within the scope of the invention and the scope limited by independent claims and dependent claims.

## Claims

1. A microfluidic device for testing an analyte in a liquid sample, comprising:

   a channel comprising an inlet and an outlet; and
   a dried reagent area and a testing area that are located in the channel, wherein the testing area is located downstream of the dried reagent area;

and the inlet of the channel is configured to receive a liquid sample.

2. The microfluidic device according to claim 1, wherein the dried reagent area is located downstream of the inlet of the channel, and the dried reagent area comprises a liquid inlet and a liquid outlet.

3. The microfluidic device according to any one of claims 1-2, wherein the capillary force of the liquid inlet of the dried reagent area is less than the capillary force of the liquid outlet, or the capillary force gradually increases from the liquid inlet to the liquid outlet of the dried reagent area.

4. The microfluidic device according to any one of claims 1-3, wherein the dried reagent area is a funnel-shaped area having two ends, the end close to the inlet of the channel is treated with a dried reagent, and the end away from the inlet is directly or indirectly connected to the testing area.

5. The microfluidic device according to any one of claims 2-4, wherein a width of the inlet of the dried reagent area is greater than a width of the outlet.

6. The microfluidic device according to any one of claims 2-5, wherein a flow rate of the liquid per unit time at the inlet of the dried reagent area is greater than a flow rate of the liquid per unit time at the outlet.

7. The microfluidic device according to any one of claims 2-6, wherein a cross-sectional area of the inlet of the dried reagent area is greater than a cross-sectional area of the outlet.

8. The microfluidic device according to any one of claims 1-7, wherein a sample channel is arranged upstream of the inlet of the channel, and capillary force of the sample channel is less than the capillary force of the inlet of the channel.

9. The microfluidic device according to any one of claims 2-8, wherein a choke area is arranged downstream of the dried reagent area, and the choke area is capable of reducing a flow velocity of the liquid in the channel from the inlet of the channel to the outlet of the dried reagent area.

10. The microfluidic device according to claim 9, wherein the choke area comprises a choke channel, and a widest distance of the choke area is greater than a maximum distance of the liquid outlet of the dried reagent area.

11. The microfluidic device according to any one of claims 1-10, wherein the device further comprises a mixing area, and the mixing area is located between the dried reagent area and the testing area.

12. The microfluidic device according to claim 11, wherein the liquid in the mixing area flows in a substantially non-laminar flow manner.

13. The microfluidic device according to any one of claims 11-12, wherein a distance or time of the liquid flowing in the mixing area is greater than a distance or time of the liquid flowing in the dried reagent area or the testing area.

14. The microfluidic device according to any one of claims 11-13, wherein the liquid flows in the mixing area along two or more flow paths.

15. The microfluidic device according to any one of claims 11-14, wherein the liquid flows at different flow velocities, for different times, for different times or in different directions in each flow path.

16. The microfluidic device according to any one of claims 11-15, wherein the liquid is gathered together after flowing through different flow paths.

17. The microfluidic device according to any one of claims 11-16, wherein the mixing area comprises a choke element, and the choke element is capable of dividing the liquid in the mixing area so as to form the two or more liquid flow paths.

18. The microfluidic device according to any one of claims 11-17, wherein the mixing area contains at least one mixing unit, each mixing unit comprises one choke element, the liquid is divided into different liquid flow paths by the choke element, and the liquid flowing through different liquid flow paths is gathered together in the mixing unit to enter the next mixing unit.

19. The microfluidic device according to any one of claims 11-18, wherein the plurality of mixing units are sequentially arranged from upstream to downstream from the outlet of the mixing area to the outlet of the mixing area.

20. The microfluidic device according to any one of claims 11-19, wherein a buffer area is arranged downstream of the mixing area, the testing area is arranged downstream of the buffer area, and the buffer area is capable of gradually reducing a flow velocity of the liquid flowing out of the mixing area.

101

105

102

106

103

104

Fig. 1

Fig. 2

Fig. 3

Fig. 4

Fig. 5

Fig. 6

Fig. 7

Fig. 8

Fig. 9

Fig. 10A

Fig. 10B

Fig. 10C

Fig. 11

1139

113-1

113-5

119

1140

113-2

113-6

113-3

113-7

113-4

114

Fig. 12

105

1135

1134

106

1136

Fig. 13

Fig. 14

*1147*

*1146*

Fig. 15

Fig. 16

115

1152

1153

Fig. 17

Fig. 18

Fig. 19

113

30''

34ul/min

34.8ul/m

114

34.5ul/m

34.6ul/m

2'52''

34.7ul/m

Fig. 20A

113

114

22.6ul/mi

22.6ul/min

22.6ul/min

22.6ul/min

22.6ul/min

3'55'

Fig. 20B

EP 4 732 950 A1

Fig. 20C

55

Fig. 21

Europäisches
Patentamt

European
Patent Office

Office européen
des brevets

# EUROPEAN SEARCH REPORT

**Application Number**

EP 25 16 6456

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/164627 A1 (BATTRELL C FREDERICK [US] ET AL) 28 June 2012 (2012-06-28) * paragraphs [0119], [0121], [0125]; figure 3 * | 1-20 | INV. B01L3/00 |
| X | CN 113 996 355 B (SHANGHAI JUNZHEN LIFE SCIENCE CO LTD) 6 June 2023 (2023-06-06) * paragraphs [0056], [0057], [0064], [0066], [0071], [0091]; claim 1; figure 2 * | 1-20 | |
| X | US 2010/112723 A1 (BATTRELL C FREDERICK [US] ET AL) 6 May 2010 (2010-05-06) * paragraphs [0178], [0181], [0183] - [0185]; figure 8 * | 1-20 | |
| X | CN 112 945 637 A (SHANGHAI JUNZHEN LIFE SCIENCE CO LTD) 11 June 2021 (2021-06-11) * paragraphs [0020], [0021], [0023]; figure 2 * | 1-20 | |

TECHNICAL FIELDS
SEARCHED (IPC)

B01L

The present search report has been drawn up for all claims

| Place of search | Date of completion of the search | Examiner |
|---|---|---|
| The Hague | 5 September 2025 | Vahidpour, Farnoosh |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

 

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 16 6456

This annex lists the patent family members relating to the patent documents cited in the above-mentioned European search report.
The members are as contained in the European Patent Office EDP file on
The European Patent Office is in no way liable for these particulars which are merely given for the purpose of information.

05-09-2025

| Patent document cited in search report | | | Publication date | Patent family member(s) | | | Publication date |
|---|---|---|---|---|---|---|---|
| US 2012164627 | A1 | | 28-06-2012 | AU | 2007265628 | A1 | 03-01-2008 |
| | | | | EP | 2041573 | A2 | 01-04-2009 |
| | | | | US | 2009181411 | A1 | 16-07-2009 |
| | | | | US | 2012164627 | A1 | 28-06-2012 |
| | | | | US | 2015158026 | A1 | 11-06-2015 |
| | | | | WO | 2008002462 | A2 | 03-01-2008 |
| CN 113996355 | B | | 06-06-2023 | NONE | | | |
| US 2010112723 | A1 | | 06-05-2010 | EP | 2349566 | A1 | 03-08-2011 |
| | | | | US | 2010112723 | A1 | 06-05-2010 |
| | | | | US | 2013142708 | A1 | 06-06-2013 |
| | | | | US | 2016209431 | A1 | 21-07-2016 |
| | | | | WO | 2010040103 | A1 | 08-04-2010 |
| CN 112945637 | A | | 11-06-2021 | CN | 112945637 | A | 11-06-2021 |
| | | | | WO | 2022161424 | A1 | 04-08-2022 |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2024114832844
- CN 2025102795425
- US 63714264
- CN 202110956861
- US 7824611 B
- US 2013035505 W
- CN 202310561939
- US 286405